# EUROPEAN PATENT APPLICATION

(11) **EP 2 067 861 A1**
(43) Date of publication of application: **10.06.2009**
(21) Application number: 07122366.3
(22) Date of filing: 05.12.2007
(51) Int. Cl.: C12N 15/10, A61K 49/00, A01K 67/027

(54) **Animals of the rattus genus wherein msh6 expression is functionally decreased and methods for the generation of specific mutants therefrom**

(71) Applicant: Koninklijke Nederlandse Akademie van Wetenschappen, 1011 JV Amsterdam (NL)
(72) Inventor: Cuppen, Edwin Pieter Johan Gerard, 3572 KG, Utrecht (NL); van Boxtel, Ruben, 2595 VD, Den Haag (NL)
(74) Representative: Hatzmann, Martin

(57) **Abstract**

The invention provides means and methods for generating animals of the rattus genus carrying mutagen induced mutations in the genome and new mutant animal models. In a preferred embodiment the invention provides a method for producing an animal of the genus rattus with a mutagen induced mutation in a germ line cell comprising providing said animal having said germ line cell with a mutation inducing amount of a mutagen, said method **characterised in that** msh6 expression in said germ line cell is functionally decreased

## Description

The invention relates to the field of genetics. More specifically, the invention relates to the use of a mutant animal in a method for performing a genomic mutagenesis screen. The invention further relates to a novel mutant animal, and to the use of an animal resulting from said mutagenesis screen as an animal model for a human deficiency.

The mismatch repair system (MMR) recognizes mismatches that are incorporated during DNA replication and surpass the proofreading activity of DNA polymerase. The MMR machinery is highly conserved in evolution and the prototypic system was first elucidated in Escherichia coli (Lahue, et al. (1989) Science 245, 160-4). In this model, a mismatch is recognized by the MutS homodimer followed by binding of the MutL homodimer and activation of the repair pathway. This leads to the excision and re-synthesis of the error containing DNA strand. In eukaryotes MutS function is separated in the recognition of single-base mismatches and small insertions or deletions loops (IDL's) of one or two extra helical nucleotides by the MutSa subunit and the recognition of large IDL's of two to four nucleotides by MutSb subunit. Both subunits are heterodimers whereby MutSa consists of MSH2 and MSH6 and the MutSb of MSH2 and MSH3 (Jiricny, J. (2006) Nat Rev Mol Cell Biol 7, 335-46).
Besides maintaining the genomic integrity during replication the MMR systems also recognizes different forms of DNA damage caused by genotoxic agents (Duckett et al. (1996) Proc Natl Acad Sci U S A 93, 6443-7). Cell lines deficient for MMR are more resistant to cell death caused by alkylating agents (Claij and Te Riele (2002) Oncogene 21, 2873-9; Claij et al. (2003) Cancer Res 63, 2062-6.), antimetabolites and intrastrand crosslinking agents (Yang et al. (2004) Cancer Cell 6, 139-50). Two hypotheses have been proposed to explain MMR mediated apoptosis. The first states that the repeated activation of MMR due to wrong nucleotide incorporation by polymerase at the site of DNA damage causes replication fork arrests, which ultimately would lead to lethality (Karran and Bignami (1992) Nucleic Acids Res 20, 2933-40).

The second hypothesis suggests that the MMR systems can function as a molecular sensor, which can directly signal the apoptotic machinery in cause of high levels of DNA damage (Fishel (1999) Nat Med 5, 1239-41).

In humans mutations in the DNA mismatch repair genes have been linked to the cause of hereditary non-polyposis colorectal cancer (HNPCC) also referred to as the Lynch syndrome, which is characterized by early-onset colon cancer (Lynch and Smyrk (1996) Cancer 78, 1149-67). Mutations in the msh6 gene have been associated with an atypical HNPCC phenotype characterized by a late onset and a high occurrence of extra colonic tumors, especially in the endometrium (Wijnen et al. (1999) Nat Genet 23, 142-4). A hallmark of MMR deficiency in HNPCC tumors is a significantly higher mutation rate, termed replication error phenotype (RER+) reflected by an elevated spontaneous mutation frequency and the instability of simple repeat length during mitosis (Ionov et al. (1993) Nature 363, 558-61). The latter phenomenon is referred to as microsatellite instability (MSI) and has proven to be a powerful tool to diagnose HNPCC tumors (Boland et al. (1998) Cancer Res 58, 5248-57).

Most of our knowledge about the MutS genes in mammals results from mouse knockout models. Complete loss of MMR recognition in the msh2-/- mouse results in a strong reduction in the survival of these mice with a median survival time of 5 - 6 months (Reitmair et al. (1996) Cancer Res 56, 3842-9). At an early stage in life these mice develop lymphomas followed by a later onset of adenomacarcinomas in the gastrointestinal tract (Reitmair et al. (1996) Cancer Res 56, 3842-9; de Wind et al. (1995) Cell 82, 321-30). Knocking out the msh6 gene in mice also results in a decreased life span, however, with a median survival time of 10 months this is less severe than for msh2-/-. The msh6-/- mouse predominantly develops lymphomas and tumors in the small intestine and several tumors in other tissues, like the liver and skin (Edelmann et al. (1997) Cell 91, 467-77). Deletion of the msh3 gene in mice did not induce a cancer phenotype, however, deletion of this gene in a Msh6 deficient background led to an indistinguishable cancer phenotype from that of the msh2-/- mouse, suggesting redundancy between Msh6 and Msh3 (de Wind et al. (1999) Nat Genet 23, 359-62). Although mouse knockout models have revealed much of mammalian MMR, one drawback in modeling human HNPCC has been the differences in tumor spectra. The human HNPCC phenotype primarily shows colorectal tumors, whereas the mouse models develop tumors in the small intestine (Reitmair et al. (1996) Cancer Res 56, 3842-9; Edelmann et al. (1997) Cell 91, 467-77). Furthermore, the high occurrence of endometrial cancers found in human atypical HNPCC, especially in the case of MSH6 germ line mutations (Wijnen et al. (1999) Nat Genet 23, 142-4), is not reflected by the mouse model, urging the need for an independent model system.

Over the last decades, the rat has matured to an important genetic model system (Lazar et al. (2005) Genome Res 15, 1717-28; Smits and Cuppen (2006) Trends Genet 22, 232-40). The complete genome sequence of the rat is now available (Gibbs et al. (2004) Nature 428, 493-521) and recently developed techniques make it possible to generate knockout rats using a target-selected N-ethyl-N-nitrosourea (ENU)-driven mutagenesis approach (Smits et al. (2006) Pharmacogenet Genomics 16, 159-69; Zan et al. (2003) Nat Biotechnol 21, 645-51).

The present methods for generating mutant rats are cumbersome. Large amounts of animals need to be screened after the introduction of alterations in the genome of a tester animal. This is mainly due to the frequency of said alterations, which are dependent on the mutagenicity of the mutagens used to introduce said alterations. An optimal alteration frequency using such animals is found to result in about 1 alteration per 1.2x10⁶ or 1,5x10⁶ base pairs. A higher amount of mutagen results in reduced fertility and ultimately enhanced lethality of the tester animal, thereby increasing the work load that is needed for isolating an alteration in a genomic region of interest.

For example, 3,000 F1 animals, resulting from crosses between mutagenized males and untreated female mice, had to be analyzed to identify an alteration that disrupts gene function in six out of about 100 specific genes of interest, while the animals had to be kept alive until the results of the analyses were known, to allow further breeding of an animal comprising said alteration. The total amount of animals that have to be screened to identify a deleterious mutation depends on the size of the gene of interest. About 60.000 F1 animals have to be screened to identify a truncating alteration due to the introduction of a stop codon in a gene of about 1.5 kilo basepairs (kb) with a probability of 95%. With the same probability, about 10.000 F1 animals are required for identification of a truncating alteration in a gene of about 7.5 kb.

The present invention provides means and method for significantly improving the generation of genetic rat models. It was found by the present inventors that a rat comprising an inactivating mutation in a Msh6 gene (Msh6-/-) strongly enhances the alteration frequency in genomic mutagenesis screens from about 1 alteration in every 1 to 1.5 megabasepairs (mbp) to about 1 alteration in every 500 kilopasepairs (kbp), which is about 2 to 3 times more efficient. An enhanced alteration frequency was observed in homozygous Msh6-/- rats. Furthermore present inventors have determined that the mutation spectrum of ENU changes in MSH6 deficient background compared to MSH6 proficient background. The percentage of G:C to A:T transitions was significantly increased in MSH6 deficient background as compared to proficient background, whereas the percentage of A:T to G:C transitions was significantly decreased. No significant change was observed for the percentages of transversions. When considering the codon usage of a representative set of rat genes, it was found that the chance of introducing a premature stop is increased by 16% (from 4.3% to 5.0%) in MSH6 deficient background. Heterozygous rats most probably would not show an increased alternation frequency, because alternations can still be recognized and repaired by available MSH2/MSH6 subunit. In vitro studies with heterozygous MSH6 mouse cell line extracts showed no significant reduction in mismatch repair efficiency (Edelmann et al. (1997) Cell 91, 467-77). In addition it was found that the homozygous mutant animals have an average life expectancy that is compatible with the mutagenesis and breeding procedure also upon administration of the mutation inducing amount of mutagen. It was found that these animals are particularly suited for the generation of genetic models.

One alteration in every 500 kilopasepairs (kbp) indicates that a rat with a genome size of 3,000,000 kbp harbors about 6.000 genomic alterations. Because the coding region (the transcriptome) of the rat encompasses about 35,000 kbp, about 70 of these alterations will reside in coding regions. On average, about 24% of these alterations are silent, about 70% result in an amino acid changes and about 6% result in the introduction of a premature stopcodon. As a consequence, on average about 50 amino acid changing alterations and 4 premature stopcodons are present in said mutagenized Msh6-/- rat. In contrast, a mutagenized wild type rat has 20 amino acid changing mutations and 1.6 premature stopcodons, while a non-treated wild type rat has on average 0.25 alterations per animal and 0.02 stopcodons per animal.

The present invention therefore provides a method for generating an animal of the genus rattus having a mutation in a germ line cell comprising providing said animal with a mutation inducing amount of a mutagen, said method characterised in that msh6 expression in said germ line cell is functionally decreased. The invention also provides a method for providing a germ line cell of an animal of the genus rattus with a mutation comprising providing an animal of said genus comprising said germ line cell with a mutation inducing amount of a mutagen, characterised in that msh6 expression in said germ line cell is functionally decreased. Further provided is a method for providing a germ line cell of an animal of the genus rattus with a mutation comprising contacting a germ line cell of said *animal in vitro* with a mutation inducing amount of a mutagen and transplanting said germ line cell into said animal or a compatible member of the same species, said method characterised in that msh6 expression in said germ line cell is functionally decreased. In a preferred embodiment a rattus of the species norvegicus is used and preferably a laboratory strain thereof. The term rat is preferably used herein to refer to the rattus species norvegicus. Preferably said animal has, on average about 50 amino acid changing alterations and 4 premature stopcodons in said mutagenized animal. In contrast, a mutagenized wild type rat has 20 amino acid changing mutations and 1.6 premature stopcodons, while a non-treated wild type rat has on average 0.25 alterations per animal and 0.02 stopcodons per animal.

Many different mutagens are currently known. Finding a mutation inducing amount is straightforward for the person skilled in the art. Preferably the optimal mutation inducing amount is determined in a dosage-response experiment. Suitable starting points for the dose response curve can be found in in vitro experiments using cell lines preferably of the species to be tested.

For other species then rattus norvegicus the dosage used in the norvegicus is used as a starting point.

The increased level of mutagen induced mutations in such a germ line cell increases the chance of finding a mutation in a gene other than msh6. Expression of msh6 can be decreased in various ways. Mutations are preferably present in the coding region, the promoter or other transcription/translation sequences. RNA coding for msh6 can be provided with antisense nucleic acid to target said RNA for destruction and/or to inhibit translation of the RNA. Other methods include the induction of exon skipping to generate non-sense coding regions. Yet other methods include the insertion of transposons or retroviral vectors that disrupt the coding region or insertions/deletion that inactivate transcription through methylation. Essential in any method for functionally decreasing msh6 expression is that the level of msh6 protein in the cell is reduced. Preferably to levels that are less than 40% of the level in the homozygous msh6⁺ rat germ line cell. Preferably, the level is even lower. Preferably less than 10%, and more preferably less than 1%. In a preferred embodiment no functional msh6 protein is detectable in the germ line cell. The same levels of msh6 protein are preferred in case essentially all cells of said animal have a functionally decreased msh6 expression. This is preferably achieved through a genetic modification that prevents any detectable production of functional msh6 protein. Such a genetic modification is termed an inactivating mutation. Thus preferably said germ line cell and/or said animal wherein essentially all cells comprise said functionally decreased msh6 expression comprises an inactivating mutation of msh6. Said genetic modification or inactivating mutation preferably comprises a modification that disrupts the coding region or inactivates the promoter of msh6. In a particularly preferred embodiment said mutation comprises the introduction of a premature stopcodon in the coding region of msh6. Preferably said stopcodon is introduced in exon 4 of msh6 as described in Smits et al (2006). A method of the invention is preferably used in a genomic mutagenesis screen.

A germ line cell is a gamete or a cell that contributes to the formation of gametes. Germ line cells are functionally defined as cells that can fix mutations in the germ line of a species. In other words, that can produce an entire animal either directly or indirectly via precursors, chimeras, or fusion of gamete (nuclei). Such cells provide a heritable genetic change in the germ line of an animal species. Such cells include but are not limited to spermatogonial stem cells. In a preferred embodiment said germ line cell is a precursor of male gametes. In line with this preference it is preferred that said animal of the genus rattus is preferably a male animal.

The term "genomic mutagenesis screen" indicates the screening of a collection of mutagenized animals or progeny thereof for the presence of specific gene alterations. A genomic mutagenesis screen comprises genome-wide screens, directed at identifying alterations in all genes, and region-specific or gene-specific screens, directed at alterations in a specific chromosomal region or gene. Said screen can be saturated, indicating that the probability that said collection comprises an alteration in a particular gene is at least 95%, or semi-saturated or non-saturated. Said mutagenesis screen may or may not be suited for targeting the DNA of specific cell organelles, like mitochondria, because it is unclear whether MMR plays a role in the DNA repair in these organelles. In vitro studies using rat liver cell extracts has shown that mismatch repair activity is present in mitochondria, however no presence of MSH2 was detected at protein level (Mason et al. (2003) Nucleic Acids Res. 31, 1052-58). This indicates that MMR in this organelle is mediated independent of the MSH2/MSH6 dimer, because MSH6 and MSH3 are unstable and rapidly degraded when the binding protein MSH2 is absent (Drummond et al. (1997) Proc. Natl Acad. Sci. USA 94, 10144-49).

In a preferred aspect of the invention, said further alteration, which is additional to the alteration in the Msh6 gene, is present in the germline of said rat. For the identification of at least one further genomic alteration, mutagenized rats, preferably male rats, are preferably crossed to non-mutagenized rats, preferably female rats, to generate F1 animals. Following routine breeding schemes, these F1 rats are further crossed to generate progeny rats that are homozygous for one of said at least one further alteration. It is furthermore preferred to mutagenize adult male rats at an age of at least 8 weeks, preferably at an age of about 12-14 weeks, and to wait a full round of spermatogenesis, about 60-70 days, before crossing the rats to non-mutagenized female rats. This will result in the occurrence of further alterations in a large proportion of the resulting F1 generation.

The enhanced alteration frequency of Msh6-/- rats results in a requirement of a reduced number of F1 animals per genome wide saturated screen. In the above described example 20.000 compared to the 50.000 wild type F1 animals. This not only reduces the number of animals and animal housing costs, but also considerably reduces total screening costs. Another advantage is the altered spectrum of modifications with a significantly higher chance for the introduction of premature stopcodons that can be obtained using a method of the invention. A further advantage of the use of a Msh6-/- rat compared to other Msh6-/- animals in a method of the invention is the late onset of spontaneous tumorigenesis in Msh6-/- rats, which starts at about 9 months, compared to at about 3 months in Msh6-/- mice. Furthermore, a Msh6-/- zebrafish (Danio rerio) was found not to result in an enhanced alteration frequency following exposure to a mutagen. The present invention for generating progeny using cells wherein Msh6-expression is functionally decreased is also suitable for the generation of plants cells having mutation as a result of exposure to a mutation inducing amount of a mutagen.

An inactivating mutation in a Msh6 gene preferably results in a functionally disabled Msh6 protein. Said mutation is any genomic or epigenomic alteration that results in the production of an abnormal, nonfunctional Msh6 protein, a partially active version of the protein, a reduced level of expression of said protein, or the absence of Msh6 protein. A functionally disabled Msh6 results in an increased number of genomic mismatches that are left unrepaired during cell division. The mismatch repair protein Msh6, in a heterodimer with Msh2, forms the mammalian MutS complex, which is part of the mismatch repair (MMR) system. The MMR system is responsible for removal of base mismatches caused by spontaneous and induced base deamination, oxidation, methylation and replication errors. The MutS complex recognizes base mismatches and forms an active complex with MLH1-PMS2 proteins for actual repair of the mismatch after recognition. Methods to determine a deficiency in the MMR system are known to a skilled person and include, but are not limited to, methods for determining microsatellite instability (MSI) in simple repeats, and methods for determining the frequency of point mutations.

In a preferred embodiment, said inactivating mutation results in a premature stop codon in the coding part of said Msh6 gene. Mutations in the human MSH6 gene have been reported in about 10 percent of hereditary nonpolyposis colon cancer (HNPCC) families. A high percentage of these mutations result in premature stop codons, causing the production of an abnormally short, nonfunctional MSH6 protein. Therefore, the presence of a premature stop codon closely resembles the frequently occurring functionally disabled human MSH6 protein.

In a most preferred embodiment, said inactivating mutation comprises the mutation of a codon for a leucine at amino acid position 306 of the protein to a stop codon. Said mutation resides between the PCNA binding domain and the mismatch-binding domain and results in nonsense-mediated decay of the mRNA transcript. The MSH6 protein consists of a (1) PCNA domain that is needed for connecting the protein to DNA replication, a (2) mismatch-binding domain, a (3) connector domain that is involved in allosteric signaling between the levers domain and the ATPase domain, the (4) levers domain, which is most probably involved in signal transduction between the ATPase and DNA binding domains, the (5) clamp domain that make significant nonspecific DNA contact and the (6) ATPase domain (Warren et al. (2007)Mol Cell 26, 579-92). Alterations that influence the structure of any of these characterized domains will most probably influence the protein function in one way or another. Premature stops in most cases will lead to nonsense-mediated decay of the mRNA and will completely abolish protein function.

A further alteration can be introduced in Msh6-/- rats by any means known to a skilled person, comprising radiation such as ionizing radiation and ultraviolet light, insertional mutagenesis, and chemical mutagenesis. A mutagen induced mutation is preferably a point mutation. Preferably a transversion or a transition.

A preferred mutagen in a method of the invention comprises a chemical mutagen.

A chemical mutagen in general causes alterations in the DNA of cells that are repaired by the MMR system. A deficiency in the MMR system will result in an enhanced alteration frequency following exposure to a chemical mutagen. Known chemical mutagens comprise a base analogue such as bromouracil and aminopurine,; an intercalating agent such as acridine orange, proflavin, and ethidium bromide; a deaminating agent such as nitrous acid; and an alkylating agent, such as a methylating or ethylating agent.

A preferred chemical mutagen comprises an alkylating agent, such as, for example, nitrosoguanidine, methyl methanesulfonate, ethyl methanesulfonate (EMS) and, most preferred, N-ethyl-N-nitrosourea (ENU).

ENU is an alkylating agent that is a powerful mutagen in spermatogonial stem cells. Therefore, the use of ENU as a chemical mutagen results in a high percentage of further alterations that are present in the germline of said mutagenized rat.

ENU predominantly modifies A:T base pairs, with 44% A:T->T:A transversions, 38% A:T->G:C transitions, 8% G:C ->A:T transitions, 3% G:C->C:G transversions, 5% A:T->C:G transversions and 2% G:C->T:A transversions, resulting in about 64% missense mutations, 10% nonsense mutations and 26% splicing errors (Justice et al. (1999) Hum Mol Gen 8: 1955-1963). Therefore, the use of ENU as a mutagen will result in about 4 to 5% of said further alterations introducing a premature stop codon as a result of a nonsense mutation.

Said chemical mutagen can be administered orally, intravenously, intraperitoneally, or directly injected into the testis. However, care is to be taken that the solvent used does not interfere with the route of administration. Furthermore, some routes of administration are not applicable for all mutagens, as is known to a skilled person. For example, ENU is not effective if orally applied to rats and is preferably administered intrapreritonally.

The use of a mutagen in an animal of the genus rattus, comprising cells wherein msh6 expression is functionally decreased, results in mutations that are different in different cells of the animal. The average mutation frequency in male germ line cells as calculated in the experimental section is markedly elevated when compared to a wild type animal. Thus the invention further provides an animal of the genus rattus comprising a cell with a mutagen induced mutation characterised in that Msh6 expression in said cell is functionally decreased. Preferably said animal of the genus rattus comprises a plurality of cells wherein msh6 expression is decreased, preferably said plurality of cells comprise different mutagen induced mutations. Preferably essentially all cells of said animal comprise a functionally decreased Msh6 expression. With essentially is meant that some cells also normally do not express msh6. For instance mature red blood cells that do no longer contain a nucleus and skin cell that stop RNA synthesis as they mature and move upward in the skin. The observed mutation frequency is higher than any mutation frequency previously observed in animals that are capable of generating offspring. An animal of the invention thus preferably comprises an inactivating mutation of msh6 in essentially all cells and at least one mutagen induced mutation in every 0.1 x 10⁶ to 1 x 10⁶ base pairs, preferably 0.5 x 10⁶ - 1 x 106, more preferably 0.1 x 10⁶ - 0.5 x 10⁶. This animal can be the mutagen treated animal, or offspring thereof. Offspring is typically generated with a normal counterpart, i.e. not exposed to a mutagen. In this offspring one set of chromosome, e.g. derived from the mutagen exposed animal comprises the mutagen induced mutations with at least the indicated frequency while the other set of chromosomes does not. In a preferred embodiment the same chromosome in each cell comprises the same (frequency) of mutagen induced mutation. Thus the present invention further provides an animal with a germ line cell that comprises at least one mutagen induced mutation in every 1 x 10⁶ base pairs on at least one set of chromosomes. Preferably said germ lines cells of said animal comprise on average at least one mutagen induced mutation in every 1 x 10⁶ base pairs. Preferably essentially all cells of said animal comprise a functionally decreased msh6 expression. An animal of the invention preferably comprises a plurality of germ line cells comprising the mentioned mutagen induced mutation, wherein at least two and preferably at least 5 germ line cells comprise different mutagen induced mutations.

Using a method of the invention, many different mutations were generated and detected in progeny rats. Many of these mutations are listed in table 7 and table 8. In a preferred embodiment in animal of the invention comprises a mutation in a gene as listed in table 7 or table 8.
The mutant of the gene listed in table 7 preferably comprises the mutation as specified in table 7. The animals can be used directly to generate a genetic model. However, typically the rat having an interesting mutation is crossed into a suitable background that typically no longer comprises the genetic modification resulting in decreased msh6 expression, although this does not necessarily need to be the case. Double or further mutants can be of interest also, for instance as a model for HNPCC.
Thus the present invention further provides progeny of an animal according to the invention, having a mutation in a gene as listed in table 7 or 8 and comprising cells having functional msh6 expression. Crossing may result in the generation of animals that are homozygous for a mutation in the gene of table 7 or 8. Such homozygotes typically, though not necessarily have the same mutation in each of the copies in the cell. Thus preferably the invention provides an animal of the invention that is homozygous for said mutation in a gene listed in table 7 or table 8. The invention further provides an animal of the genus rattus comprising a cell with a mutagen induced mutation which animal is a progeny of an animal according to the invention. Preferably said animal comprises a cell wherein msh6 expression is functionally decreased.

The identification of said at least one further alteration in progeny of a mutagenized animal can be performed according to any method known in the art. Phenotypic screens can be performed for the identification of phenotypic alterations in progeny of said mutagenized animal. The presence of a dominant alteration that results in a visible phenotype may be scored already in the F1 generation, while a breeding scheme allows scoring for the presence of a recessive alteration including a developmental lethal alteration. A phenotype that, for example, affects the gross anatomy, the coat, or the size of an animal can be easily identified. Furthermore, X-ray analysis, blood tests, and/or specific tests such as behavioral tests, learning test and/or memory test can be introduced in the screening program to identify alterations that affect the outcome of these tests. In addition, microarray analyses can be used to identify changes in gene expression patterns in a specific tissue of during a specific developmental stage.

A breeding scheme can also be applied to identify modifiers of a known pathway by introducing a rat model of a human disease and identifying genomic alterations that result in an amelioration or cure of said model disease. Alternative, a marker gene can be introduced in the breeding scheme and the progeny rats can be screened for alterations that modify, for example, the expression or localization of said marker gene.

Further to the phenotypic screens, or in addition thereto, gene- or region-specific screens can be performed to isolate alterations in a specific gene or genes, or in one or more genomic regions. Following the isolation of genomic DNA from a progeny of the mutagenized animals, the genomic regions of interest can be amplified, for example by cloning, polymerase chain reaction (PCR) and/or multiplex PCR, and an alteration in the amplified material, as compared to the progeny of a non-mutagenized rat, can be determined. Methods for high-throughput single nucleotide polymorphism detection are known in the art, including DNA resequencing techniques, conformation-sensitive capillary electrophoresis, high-resolution "melt" analysis, sequence analysis such as array-based sequence analysis. Targeting induced local lesions in genomes (TILLING) approaches can be used for combining chemical mutagenesis with a sensitive DNA screening-technique in a target gene such as, for example, HPLC or the restriction enzyme Cel-I combined with a gel based system to identify mutations.

The invention also provides the use of a rat comprising an inactivating mutation in a Msh6 gene, in a genomic mutagenesis screen. The use of said rat will result in an enhanced alteration frequency, enabling a reduced number of animals for said screen. Said mutation preferably results in a functionally disabled Msh6 protein such as the introduction of a premature stop codon in the coding part of said Msh6 gene. A preferred mutation is a mutation of a codon for a leucine at amino acid position 306 of the protein to a stop codon.

In a further embodiment, the invention provides a male rat, or progeny there from, comprising an inactivating mutation in a gene encoding Msh6, said rat being exposed to an effective dose of a mutagen. It is preferred that said rat comprises at least one further alteration in the germ line as compared to a litter mate that was not exposed to said mutagen. Methods for determining said at least one further alteration are known in the art, including DNA resequencing, conformation-sensitive capillary electrophoresis, high-resolution "melt" analysis and sequence analysis.

The invention further provides sperm that is isolated from a rat of the invention or male progeny derived there from. Methods for isolating sperm from rats are known to a skilled person. For example, sperm can be directly isolated from the caudal epididymides or vas deferens from an anesthetized male. If required, sperm cells such as spermatozoa can be isolated from said sperm. Sperm or sperm cells can be preserved by cryopreservation applying cryoprotection methods to prevent damage during freezing and thawing. Alternatively, freeze-drying or desiccation by rapid convective drying (McGinnis et al. (2005) Biol Repro 73, 627-633) can be applied for storage of rat sperm or sperm cells.

The invention furthermore provides the use of sperm of the invention for identifying at least one further genomic alteration in progeny derived from said sperm. Sperm according to the invention can be used to fertilize rat oocytes to establish progeny from said sperm. If required, said stored sperm can be injected into oocytes by the intracytoplasmic sperm injection technique (ICSI) to achieve fertilization.

The invention also provides the use of in vitro mutagenesis of spermatogonial stem cells (SSC) or embryonic stem (ES) cells that have reduced MSH6 function, e.g. by derivation of such cells from Msh6-/- animals. Methods for isolating SSC or ES cells are known to a skilled person. SSC and ES cells can be mutagenized and cryopreserved or transplanted to recipient male testis or blastocysts, respectively. In the first case F1 progeny can be produced by breeding with a wild-type female, in the second case the injected blastocyst can be injected in recipient uterus and give rise to chimaeras, which can be bred with wild-type animals to preserve alternations.

In another aspect, the invention provides a method of selecting a rat homozygous for one of said at least one further mutation, comprising breeding a rat according to the invention or using sperm according to the invention, and selecting a rat comprising a wildtype Msh6 gene and being homozygous for one of said at least one further mutation.

A rat according to the invention comprises an inactivating mutation in a Msh6 gene and at least one further alteration. It is possible that the phenotype of one of said further mutations, either heterozygous or homozygous, is affected by said mutation in a Msh6 gene and/or one of said further alterations. Said method of the invention allows the generation of a heterozygous or homozygous rat that is mutant for only one of said further alterations, in the absence of an inactivating mutation in a Msh6 gene.

In a preferred embodiment said further alteration is an alteration in a gene as listed in table 7 or table 8. The mutation is preferably an inactivating mutation. Preferably a mutation in the coding region of said gene of table 7 or table 8. Preferably said alteration is in a gene as listed in table 7. Preferably said alteration is an alteration as listed in table 7.

The invention in addition provides the use of a rat comprising an inactivating mutation in a gene encoding Msh6 as an animal model for HNPCC. Said rat is characterized by microsatellite instability in simple repeats and point mutation instability, has a reduced life span, and develops tumors in the HNPCC spectrum. In contrast to established mouse Msh6 knockout models, said Msh6-/- rat lacks intestinal tumors, thereby more closely resembling the spectrum of tumors seen in human HNPCC patients. The Msh6-/- rat does not develop colorectal tumors but develops an atypical HNPCC spectrum of tumors, especially characterized by the presence of endometrial cancers.

The invention in addition provides the use of a rat according to the invention for testing a potential pharmaceutical therapeutic agent. The extended range of phenotypic characteristics, combined with the size of the animal, the prolonged viability and the capacity to bear big sized tumors make this rat an attractable model for specific experimental manipulations, such as for example local irradiation experiments for studying and treating residual cancer cells.

The invention further provides a method for providing a rat cell with a mutation comprising exposing said rat cell to a mutation-inducing amount of a mutagen, said method characterized in that Msh6 expression in said rat cell is functionally decreased. Preferably said rat cell is a germ line cell or derivative thereof. As mentioned herein above, embryonic or spermatogonial stem cells can be exposed in vitro, to a mutagen inducing amount of a mutagen, and be used to generate a rat, for instance via insertion into a treated blastocyst or testis. The ES cells can also be characterised for the presence of the appropriate mutation before generating said animal. The ES cell can be generated in the traditional way or be generated by dedifferentiation from a differentiated cell. For instance, it is possible to generate ES like cells from adult cells through the expression therein of three or four genes. Another method is the use of a spermatogonial stem cell. A derivative of a germ line cell is preferably a cell that is the result of transfer of a nucleus of said rat cell into a rat germ line cell.The invention further provides a rat cell comprising a mutagen induced mutation that is obtainable by a method of the invention. Said rat cell preferably comprises a genetic modification resulting in a functionally decreased expression of msh6. Preferably said rat cell is a germ line cell or a derivative thereof.
The invention further provides a collection of rat cells characterized in that said collection comprises at least one cell obtainable by a method according to the invention. Said rat cells are preferably present in a plurality of receptacles. Said cells can be used a source for screening for a desired mutation. Keeping cell in storage is more practical than maintaining unidentified mutations in life rats. Thus preferably said collection is used for identifying a mutation that is induced by said mutagen. In a preferred embodiment said collection is stored in a storage means. Preferably a storage means for long term storage such as storage in frozen form at reduced temperatures such as -70 degrees Celsius. The invention further provides the use of a rat cell or a nucleic acid containing derivative thereof from a collection of the invention for the generation of a rat.

In a further aspect the invention provides a collection of germ lines cells or derivatives thereof comprising a mutagen induced mutation and comprising an msh expression that is functionally decreased. Preferably said collection comprises a plurality of germ line cells or derivatives thereof comprising at least two and preferably at least 5 germ line cells or derivatives thereof with a different mutagen induced mutation.

### Figure legends

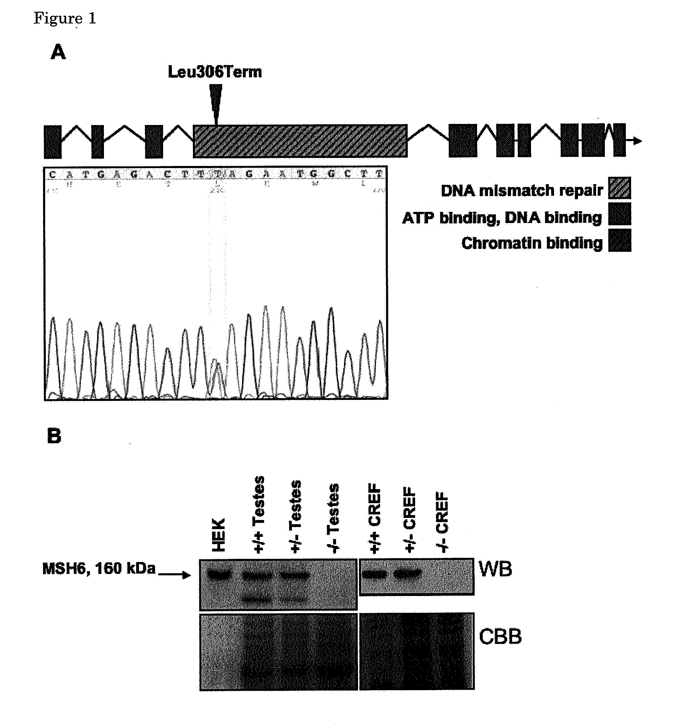

**Figure 1****.** Characteristics of the *msh6*^{*-*/*-*} rat. (A) Genomic organization of the *msh6* gene in rats. The arrow marks the position of the ENU-induced mutation that results in a premature stop codon. The sequence trace indicates the T to A transition in a heterozygous rat. (B) Western blot (WB) of testes and cultured rat embryonic fibroblast (CREF) lysates stained with antibodies against human MSH6 (160 kDa). Lysate of human embryonic kidney (HEK) cells was loaded as a positive control. MSH6 protein is completely absent in *msh6*^{*-*/*-*} testes and CREF. Coomassie brilliant blue staining (CBB) was used as a control for protein loading.

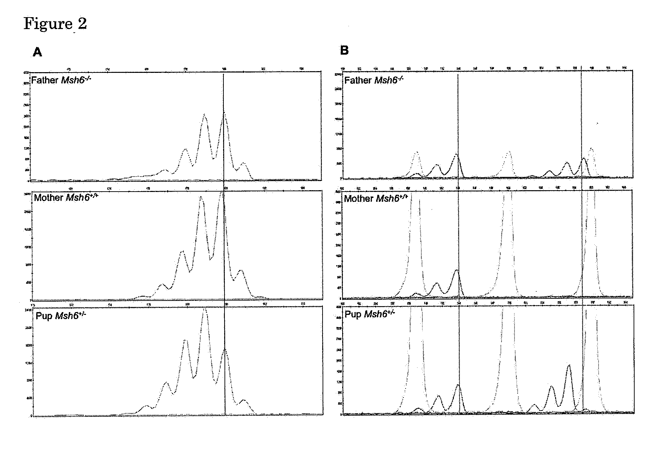

**Figure 2****.** Microsatellite instability in the germ line. (A) A mononucleotide repeat of 20 repetitive subunits (G)₂₀ was analyzed in a cross between a *msh6*^{*-*/*-*} father and a wild type mother and their *msh6*^{*+*/*-*} offspring. The red line indicates the size of the most prominent amplification product in both parents. In the DNA sample of the offspring one of the alleles has lost one repetitive subunit. (B) Fragment analysis of a dinucleotide repeat (CA)₃₆ that is polymorphic between the founder animals. As a result, progeny is heterozygous for this marker and the depicted F1 animal shows a deletion of one repetitive subunit in the larger allele, which was passed on by the *msh6*^{*-*/*-*} father. The orange peaks represent the size marker that was loaded to determine the size of the PCR product.

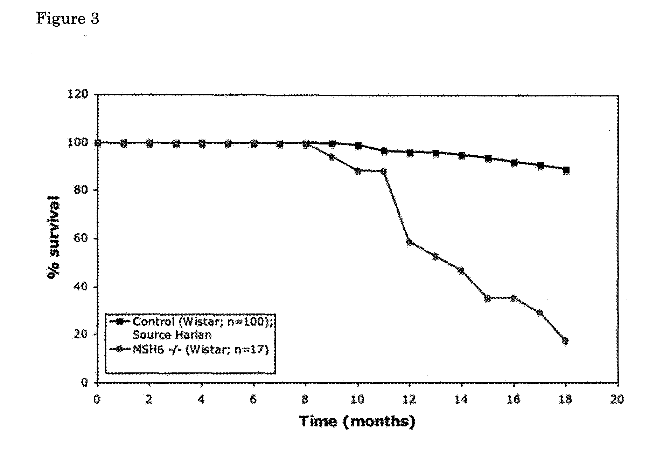

**Figure 3****.** Survival of the *msh6*^{*-*/*-*} rats. The time the rats became moribund is recorded. Red line, *msh6*^{*-*/*-*} rats (n=17); black line, control group (n=100, Source Harlan).

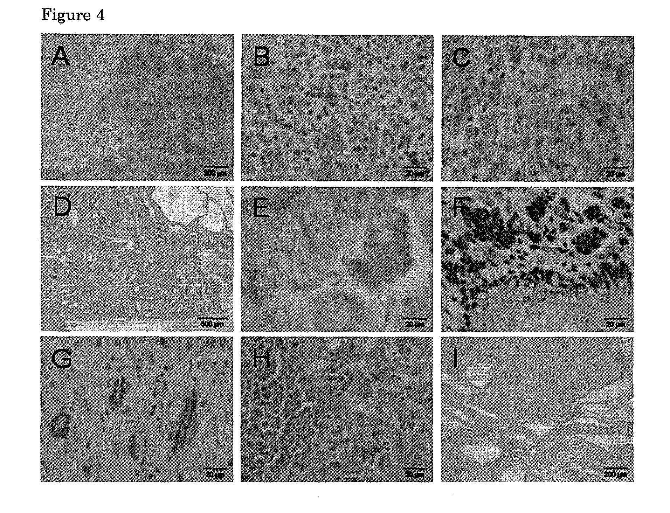

**Figure 4****.** Tumors observed in the *msh6*^{-/-} rat. (A) Section of a mediastinal lymphoblastic lymphoma of a *msh6*^{*-*/*-*} male rat stained with HE. (B) A higher magnification of the lymphoblastic lymphoma. (C) Section of a leiomyosarcoma of the endometrium stained with HE. (D) Section of an endometrial carcinoma stained with HE. (E) A higher magnification of the endometrial carcinoma. (F) A section of a squamous cell carcinoma in the stomach of a *msh6*^{*-*/*-*} female rat stained with HE. (G) Section of a fibroadenoma of a *msh6*^{*-*/*-*} male rat stained with HE. (H) Section of a liver with lymphoblastic lymphoma leukemia stained with HE. (I) Section of a testicle containing a Leydig cell tumor stained with HE.

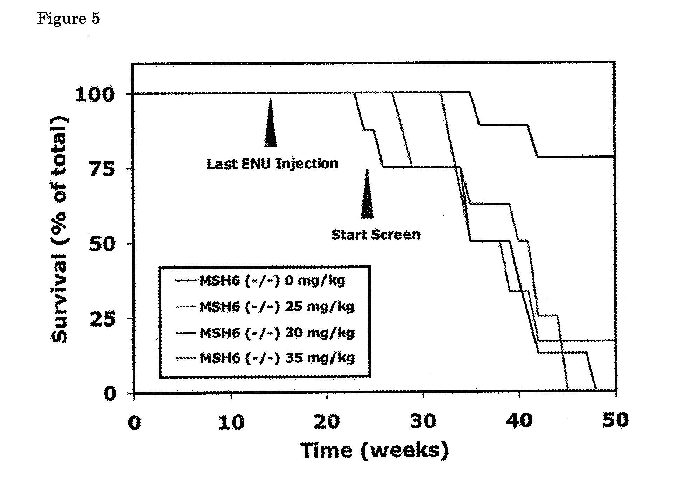

**Figure 5****.** Survival of *msh6*^{*-*/*-*} male rats after treatment of different concentrations of ENU compared to untreated *msh6*^{*-*/*-*} male rats. Time points of the last ENU injection and the beginning of the mutation discovery screen are indicated.

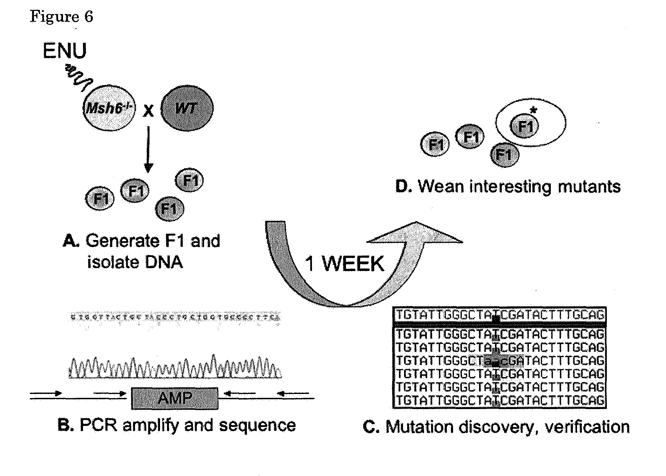

**Figure 6****.** ENU-induced target-selected knockout protocol. (A) *Msh6*^{*-*/*-*} males were mutagenized and crossed with wild-type Wistar females. At two weeks of age tissue samples from the F1 progeny were taken and DNA was isolated. (B) For every DNA samples 768 pre-selected amplicons were PCR amplified and dideoxy sequenced. (C) Sequence traces for the same amplicon of the different animals was aligned, processed with PolyPhred (Nickerson et al, (1997) Nucleic Acids Res 25, 2745-51) and analyzed using in house developed software. (D) ENU-induced de novo mutations were verified by independent PCR amplification and sequencing and pups carrying interesting mutations were weaned. The whole procedure from taking tissue samples to weaning interesting mutants takes 1 week, indicated in the centre of the figure.

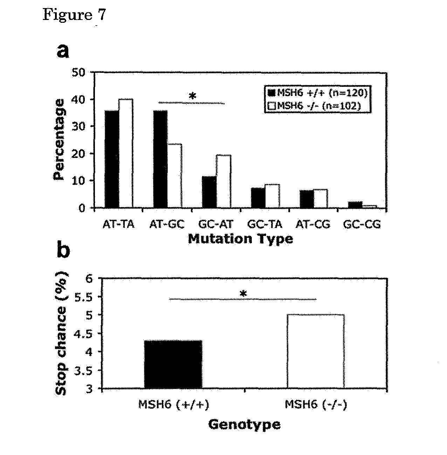

**Figure 7****.** ENU-induced mutation spectrum in MSH6 deficient background compared to MMR proficient animals. (A) The spread of ENU-induced transitions in MSH6 proficient and deficient background differs significantly, but the spread of ENU-induced transversions in MSH6 proficient and deficient background does not differ. (B) The change of introducing a premature stop codon with ENU-induced MSH6 proficient and deficient mutation spectra. For the calculations the sequences of the 768 representative exon-containing amplicons were used (* indicates significance, p < 0.05).

### Examples

### Example 1

### Materials and Methods

### Animals

All experiments were approved by the Animal Care Committee of the Royal Dutch Academy of Science according to the Dutch legal ethical guidelines. Experiments were designed to minimize the number of required animals and their suffering. The Msh6 knockout rat (Msh6^{1Hubr}) was generated by target-selected ENU-driven mutagenesis (for detailed description, see (Smits et al, (2006) Pharmacogenet Genomics 16, 159-69). Briefly, high-throughput resequencing of genomic target sequences in progeny from mutagenized rats revealed an ENU-induced premature stop codon in exon 4 of the Msh6 gene in a rat (Wistar/Crl background). The heterozygous mutant animal was outcrossed at least three times to eliminate confounding effects from background mutations induced by ENU. To obtain homozygous animals the heterozygous offspring was crossed in. At three weeks of age ear cuts were taken and used for genotyping. Genotypes were reconfirmed after experimental procedures were completed. Animals were housed under standard conditions in groups of two to three per cage per gender under controlled experimental conditions (12-h light/dark cycle, 21±1°C, 60% relative humidity, food and water ad libitum).

### Western Blot analysis

Proteins were extracted by adding lysis buffer (1% SDS, 1.0mM sodium ortho-vanadate, 10mM Tris pH 7.4) to approximately 0.25 g of tissue or cultured cells. After incubating the homogenate at 100°C for 1 min the supernatant fluids were obtained after 5 min of centrifugation at 14,000 X g at room temperature. One volume of SDS loading buffer (125 mM TRIS-CL pH 6.8, 3% SDS, 10% glycerol, 10 mM DTT and 0.1% bromophenol blue) to one volume of lysate and then incubated for 5 min at 100°C. The protein was separated on a SDS gel (6% acrylamide gradient, Bio-Rad) and transferred to a nitrocellulose membrane. Non-specific, protein-binding sites were blocked by incubating the membrane with blotting buffer: 0.05% PBS-Tween containing 5% nonfat dry milk for 1 hour at room temperature. The membrane was washed with 0.05% PBS-Tween and incubated overnight at 4°C with a 1:100 dilution of a monoclonal mouse anti-human MSH6 antibody (BD Biosciences Pharmingen) in blotting buffer. The membrane was washed five times for 10 min in 0.05% PBS-Tween and then incubated for 2 hours with peroxidase-conjugated, antimouse IgG diluted 1:2500 in blotting buffer at room temperature. The membrane was washed five times with 0.05% PBS-Tween and protein bands were detected by using the enhanced chemiluminescence detection method (ECL, Amersham Biosciences). Cell lysate from human HEK cells was used as a control and total protein was measured by Coomassie Brilliant Blue staining.

### Genotyping

Lysis on ear cuts was done overnight at 55°C in 400 µl lysis buffer, containing 100 mM Tris-HCL (pH 8.5), 200 mM NaCl, 0.2% SDS, 5 mM EDTA and 100 µg/ml of freshly added Proteinase K. Proteinase K was inactivated by incubating 10 minutes at 95°C, followed by cooling on ice. Genomic DNA was isolated by adding one volume of isopropanol, mixing and centrifugation for 40 minutes at 3000 x g at 4°C. The supernatant was discarded and the samples were rinsed with 70% ethanol and dissolved in 500 µl H2O. Genotyping was performed using the KASPar SNP Genotyping System (KBiosciences, Hoddesdon, UK) and gene specific primers (forward common, CAG TGG ACC CAC TAT CTG GTA; reverse a1, GAA GGT GAC CAA GTT CAT GCT CTT CTC TGG CTT AAG CCA TTC TA; reverse a2, GAA GGT CGG AGT CAA CGG ATT CTC TTC TCT GGC TTA AGC CAT TCT T). Shortly, a PCR was carried out using the optimal thermocycling conditions for KTaq (94°C for 15 min; 20 cycles of 94°C for 10 sec, 57°C for 5 sec, 72°C for 10 sec; followed by 18 cycles of 94°C for 10 sec, 57°C for 20 sec and 72°C for 40 sec; GeneAmp9700, Applied Biosystems, Foster City CA, USA). The PCR reaction contained 2 µl DNA solution, 1 µl 4X Reaction Mix, 165 nM reverse Primer a1 and a2 and 412.5 nM of the common forward primer, 0.025 µl KTaq polymerase and 0.4 mM MgCl2 in a total volume of 4 µl. Samples were analyzed in a PHERAstar plate reader (BMG Labtech) and data was analyzed using Klustercaller software (KBiosciences). All genotypes were confirmed in an independent reaction.

### MSI analysis

A simple repeat containing a repetitive stretch of (CA)40 (chr14:85120966-85121141, RGSC 3.4) was amplified using the following primers: 5'-6FAM-TTC AAC CAC AAT CTC GAC AG -3' (forward) and 5'- AGG CAT GAG TTC TGA GGT TC -3' (reverse); for the simple repeat (contig) with the stretch of (CA)36 (chr13:105939209- 105939433) was amplified using the following primers: 5'-6FAM- TGG CAC AGG TGT TTA GTG TC -3' (forward) and 5'-TGC AGA AGA AAT GAG AGG TG -3' (reverse); for amplifying the simple repeat containing a (G)20 (chr3:105633672-105633851) 5'-VIC- CAT TCT GGA AGT GAC TGC TG -3' and 5'- TCC ACG ATA CTG CAA TTC TC (reverse) were used and the simple repeat containing the stretch of (A)30 (chr8:118654926-118655555) was amplified using the following primers 5'-NED- GCC CTC TTC TGG TGT ATC TG -3' (forward) and 5'- AGC TTC ATC CGT TAG TGT GG -3' (reverse). The PCR was carried out using a touchdown thermocycling program (94°C for 60 sec; 15 cycles of 92°C for 30 sec, 65°C for 30 sec with a decrement of 0.6°C per cycle, 72°C for 60 sec; followed by 30 cycles of 92°C for 30 sec, 58°C for 30 sec and 72°C for 60 sec; 72°C for 180 sec; GeneAmp9700, Applied Biosystems). The PCR reaction contained ∼100 ng DNA, 0.2 µM of each forward primer and 0.2 µM of each reverse primer, 400 µM of each dNTP, 25 mM tricine, 7.0% glycerol (w/v), 1.6% dimethyl sulfoxide (DMSO) (w/v), 2 mM MgCl2, 85 mM ammonium acetate pH 8.7 and 0.4 U Taq Polymerase in a total volume of 10 µl. All the PCR products were tested on a 1% agarose gel containing ethidium bromide for the presence of the proper PCR fragment. 0.5 µl of GeneScanTM-500 LIZTM size standard (Applied Biosystems) was added to every PCR product and diluted to an end volume of 10 µl with H2O. The samples were analyzed on a 96-capillary 3730XL DNA analyzer (Applied Biosystems), using the fragment analysis protocol on 36 cm array. Simple repeat sizes were analyzed for the presence of heterozygous size polymorphism using GeneMapper software (Applied Biosystems). All samples were analyzed twice in independent PCR reactions and fragment analyses.

### Point mutation instability analysis

768 pre-selected amplicons were amplified using a nested PCR setup. The first PCR was carried out using a touchdown thermocycling program (94°C for 60 sec; 15 cycles of 92°C for 30 sec, 65°C for 30 sec with a decrement of 0.6°C per cycle, 72°C for 60 sec; followed by 30 cycles of 92°C for 30 sec, 58°C for 30 sec and 72°C for 60 sec; 72°C for 180 sec; GeneAmp9700, Applied Biosystems). The PCR reaction contained about 100 ng DNA, 0.2 µM of each forward primer and 0.2 µM of each reverse primer, 400 µM of each dNTP, 25 mM tricine, 7.0% glycerol (w/v), 1.6% dimethyl sulfoxide (DMSO) (w/v), 2 mM MgCl2, 85 mM ammonium acetate pH 8.7 and 0.2 U Taq Polymerase in a total volume of 5 µl. After thermocycling, the PCR1 reactions were diluted with 20 µl H2O and 1 µl was used as template for the second round of PCR. The second PCR was done using a standard thermocycling program (94°C for 60 sec; 35 cycles of 92°C for 20 sec, 58°C for 30 sec and 72°C for 60 sec; 72°C for 180 sec; GeneAmp9700, Applied Biosystems). PCR2 mixes contained 1 µl diluted PCR1 template, 0.1 µM forward primer, 0.1 µM reverse primer, 100 µM of each dNTP, 25 mM tricine, 7.0% glycerol (w/v), 1.6% DMSO (w/v), 2 mM MgCl2, 85 mM ammonium acetate pH 8.7 and 0.1 U Taq Polymerase in a total volume of 5 µl. Several samples of each plate were tested on a 1% agarose gel containing ethidium bromide for the presence of the proper PCR fragment. PCR2 products were diluted with 20 µl H2O and 1 µl was directly used as a template for the sequencing reactions. Sequencing reactions contained 0.1 µl BigDye (v3.1; Applied Biosystems), 1.9 µl BigDye Dilution Buffer (Applied Biosystems) and 0.4 µM of the forward primers used for the PCR2 reaction in a total volume of 5 µl. The thermocycling program that was recommended by the manufacturer was used for the sequence reactions (40 cycles of 94°C for 10 sec, 50°C for 5 sec and 60°C for 120 sec). Sequencing products were purified by ethanol precipitation in the presence of 40 mM sodium-acetate and analyzed on a 96-capillary 3730XL DNA analyzer (Applied Biosystems), using the standard RapidSeq protocol on 36 cm array. Sequences were analyzed for the presence of heterozygous mutations using PolyPhred (26) and in-house developed software. All candidate mutations were verified in independent PCR and sequencing reactions.

### Analysis of tumors

Animals were scarified by CO2/O2 suffocation. Tumors, if found, and organs including the gastrointestinal tract, lungs, liver, kidneys, spleen and thymus were removed and fixed in phosphate buffered 4% formaldehyde. Representative tissues from the tumors and organs were processed and embedded in paraffin. All tissues were prepared for Hematoxylin and Eosin stain.

### Results

### Generation of the Msh6 knockout rats

In a large ENU-driven target-selected mutagenesis screen we identified a rat carrying a heterozygous mutation in the Msh6 gene (Smits et al, (2006) Pharmacogenet Genomics 16, 159-69). The mutation, a conversion of a T into an A in exon 4, resulted in a premature stop codon at position 306 (Figure 1A). After outcrossing the mutant rat, heterozygous offspring was used to obtain homozygous mutant rats, which occurred in a Mendelian fashion (not shown). The homozygous mutant rats were viable and showed normal growth and fertility. Confirmation of the Msh6 gene knockout phenotype at the molecular level was established by Western blotting, showing that the full-length Msh6 protein of approximately 160 kDa in the testes of Msh6+/+ and Msh6+/- males was completely lacking in the testes of Msh6-/- males (Figure 1B). We could not detect any truncated protein, suggesting that either the mRNA with the premature stop codon or the truncated protein are unstable, due to nonsense-mediated decay or protein folding defects, respectively. Cell lysates of cultured rat embryonic fibroblasts also showed complete absence of Msh6 in homozygous mutant cultures, further confirming these results.

### Msh6 deficient rats show microsatellite instability in the germ line

One of the roles of Msh6 in the MMR pathway is the recognition of small insertions or deletions loops (IDL's) of one or two extra helical nucleotides (Jiricny (2006) Nat Rev Mol Cell Biol 7, 335-46). Deletion of Msh6 is therefore expected to result in the inability to repair simple sequence length polymorphisms that occur during DNA replication and result in microsatellite instability (MSI) at mono- and dinucleotide repeats. MSI was analyzed in the out crossed offspring of 9 Msh6-/- males and 19 Msh6+/+ males by assaying two mononucleotide repeats ((G)20 and (A)30) and two dinucleotide repeats ((CA)36 and (CA)40). In both mononucleotide repeats (Figure 2A) and dinucleotide repeats (Figure 2B) MSI was observed as a mono-allelic change of the length of the tested repeat. Although for some of the repeats the paternal (-/-) and maternal (+/+) contribution could not be distinguished (Figure 2A), in other cases the size of the repeat was polymorphic within the strain and showed that the MSI phenotype is exclusively contributed by the paternal allele (Figure 2B). In total 168 progeny of the 9 homozygous mutant males were tested and revealed that germ line MSI occurred in all males and in all the repeats tested (6%, ± 4% for (CA)36; 9%, ± 2% for (CA)40; 8%, ± 2% for (G)20; 10%, ± 7% for (A)30; see table 1). None of the 100 offspring samples from 19 control wild-type males and females showed MSI.

### Msh6 knockout rats show a germ line mutator phenotype

Besides recognizing length polymorphisms in simple repeats the Msh6 protein is also involved in the recognition of single-base mismatches introduced during DNA replication. The inability to perform this particular MMR function will result in a higher spontaneous germ line mutation rate. It is known that the mammalian germ line mutation frequency is around 1 x 10⁻⁸ per bp per generation (Drake et al, (1998) Genetics 148, 1667-86). By high-throughput resequencing of preselected amplicons, followed by heterozygous mutation discovery in DNA samples from the offspring of 2 homozygous males and wild-type females, the spontaneous single base pair mutation rate in the male germ line of the Msh6-/- rat was determined. In a total of 8.3 x 10⁶ resequenced base pairs 3 mutations were found (twice a C>T and once a G>A transition), indicating that the spontaneous germ line mutation rate is about 3.1 x 10-7 per bp (Table 2), which is an increase of more than 30-fold. The same amplicons were sequenced in DNA samples from the offspring of 4 wild-type animals (littermates of the Msh6-/- animals) and no mutation was found in the 6.6 x 10⁶ sequenced base pairs.

### Msh6 knockout rats show a reduced life span and develop tumors

Mouse studies have shown that Msh6 deficiency results in a reduced life span due to the development of different types of tumors (Edelmann et al. (1997) Cell 91, 467-77). We monitored 9 homozygous males and 8 homozygous females and the time they became moribund was recorded. The homozygous mutant rats showed a median survival time of 14 months, whereas 95% of wild type rats normally survive at this age (Figure 3). After 18 months all the Msh6-/- rats had become moribund and there was a tumor incidence of about 88%. These results are consistent with the observation that Msh6-deficient mice show a reduced life span although they exhibited a median survival time of only 10 months (Table 4).
Each rat was subjected to a complete necropsy and histopathological analysis. Tumor occurrence started at 9 months of age in a number of different locations and the first affected rats were males. Representative histological specimens of the tumor spectra that were observed are shown in figure 4 and the complete data are summarized in table 3. Macroscopical examination revealed a significantly enlarged spleen, which was the result of infiltration of lymphomas. In total 8/17 rats developed highly invasive lymphomas in the spleen, liver, kidney, lung and mediastinum (Figure 4). Histological analysis of the lymphomas revealed that neoplastic lymphocytes were organized in sheets separated by fine fibrovascular stroma. The round cells were uniform and of medium size with scant cytoplasm and round to ovoid nuclei containing fine chromatin and occasionally a prominent nucleolus located centrally (lymphoblastic lymphoma). On average there was one mitotic figure per 40 x high power field. Sheets of neoplastic cells infiltrate into the surrounding tissue and occasionally into vascular structures. Multiple areas within the neoplasm show apoptosis characterized by pyknotic and fragmented nuclei and bright eosinophilic cytoplasm (starry sky pattern). To ascertain the cellular origin of the lymphomas, sections of tumors were stained with CD79, a B-cell specific antibody and a CD3 antibody that is T-cell specific. Of these, 7 were determined to be B-cell lymphoblastic lymphoma and one a T cell lymphoblastic lymphoma.
Other tumors found in male *msh6*^{*-*/*-*} rats included a testicular leydig cell tumor, and a mammary fibroadenoma and adenocarcinoma. Four females suffered from vaginal bleeding and histological evaluation revealed the presence of endometrial carcinomas (Figure 4D) in three animals and uterine leiomyosarcoma in one female (Figure 4C). One female developed a gastric squamous cell carcinoma (Figure 4F). Notably, some of the tumor reached big sizes, like some of the lymphomas that had an estimated diameter in excess of 3 cm. Remarkably, the leiomyosarcoma in the uterus reached a diameter of ±5 cm and also the gastric tumor reached a big size with a diameter of approximately 2 cm (Table 3).

### Example 2

### Materials and Methods

### Animals and ENU mutagenesis protocol

All experiments were approved by the Animal Care Committee of the Royal Dutch Academy of Science according to the Dutch legal ethical guidelines. Experiments were designed to minimize the number of required animals and their suffering. Male MSH6 knockout rats (Msh6^{1Hubr}) of 12 weeks of age were given three weekly intraperitoneal injections of 25, 30 and 35 mg/kg bodyweight ENU. Preparation of ENU (Isopac; Sigma) was done as decribed (for detailed description, see (Smits et al. (2006) Pharmacogenet Genomics 16, 159-69) After the treatment the injected males were monitored for fertility by breeding with one or two females. Pups from these early matings were counted, but not analyzed. Ten weeks after the last injection, fertile males of the highest-dosed fertile group were kept on a weekly breeding scheme with two females to produce F1 progeny for mutational analysis.
Animals were housed under standard conditions in groups of two to three per cage per gender under controlled experimental conditions (12-h light/dark cycle, 21±1°C, 60% relative humidity, food and water *ad libitum*).

### Genomic DNA isolation

At two weeks of age F1 progeny were uniquely tagged by fetching ear clips. Ear clips were lysed for at least 2 hours at 55°C and 1400 rpm in a Thermomixer comfort (Eppendorf) in 400 µl lysisbuffer (100 mM Tris (pH 8.5), 200 mM NaCl, 0.2% SDS, 5 mM EDTA and 100 µg/ml of freshly added Proteinase K) followed by phenol/chloroform (1:1, vol/vol) extraction. The DNA was precipitated by adding 300 µl isopropanol, mixing and centrifuging for 20 min, at 21,000 g at 4°C. The supernatant was discarded and pellets were washed with 70% ethanol and dissolved in 500 µl MQ.

### PCR and sequencing conditions

768 pre-selected amplicons were amplified using a nested PCR setup. The first PCR was carried out using a touchdown thermocycling program (94°C for 60 sec; 15 cycles of 92°C for 30 sec, 65°C for 30 sec with a decrement of 0.6°C per cycle, 72°C for 60 sec; followed by 30 cycles of 92°C for 30 sec, 58°C for 30 sec and 72°C for 60 sec; 72°C for 180 sec; GeneAmp9700, Applied Biosystems). The PCR reaction contained ±100 ng DNA, 0.2 µM of each forward primer and 0.2 µM of each reverse primer, 400 µM of each dNTP, 25 mM tricine, 7.0% glycerol (w/v), 1.6% dimethyl sulfoxide (DMSO) (w/v), 2 mM MgCl₂, 85 mM ammonium acetate pH 8.7 and 0.2 U Taq Polymerase in a total volume of 5 µl. After thermocycling, the PCR1 reactions were diluted with 20 µl H₂O and 1 µl was used as template for the second round of PCR. The second PCR was done using a standard thermocycling program (94°C for 60 sec; 35 cycles of 92°C for 20 sec, 58°C for 30 sec and 72°C for 60 sec; 72°C for 180 sec; GeneAmp9700, Applied Biosystems). PCR2 mixes contained 1 µl diluted PCR1 template, 0.1 µM forward primer, 0.1 µM reverse primer, 100 µM of each dNTP, 25 mM tricine, 7.0% glycerol (w/v), 1.6% DMSO (w/v), 2 mM MgCl₂, 85 mM ammonium acetate pH 8.7 and 0.1 U Taq Polymerase in a total volume of 5 µl. Several samples of each plate were tested on a 1% agarose gel containing ethidium bromide for the presence of the proper PCR fragment. PCR2 products were diluted with 20 µl H₂O and 1 µl was directly used as a template for the sequencing reactions. Sequencing reactions contained 0.1 µl BigDye (v3.1; Applied Biosystems), 1.9 µl BigDye Dilution Buffer (Applied Biosystems) and 0.4 µM of the forward primers used for the PCR2 reaction in a total volume of 5 µl. The thermocycling program that was recommended by the manufacturer was used for the sequence reactions (40 cycles of 94°C for 10 sec, 50°C for 5 sec and 60°C for 120 sec). Sequencing products were purified by ethanol precipitation in the presence of 40 mM sodium-acetate and analyzed on a 96-capillary 3730XL DNA analyzer (Applied Biosystems), using the standard RapidSeq protocol on 36 cm array. Sequences were analyzed for the presence of heterozygous mutations using PolyPhred (Nickerson et al. (1997) Nucleic Acids Res. 25, 2745-51) and in-house developed software. All candidate mutations were verified in independent PCR and sequencing reactions.

### Project management and primer design using LIMSTILL

All resequencing projects were managed using LIMSTILL, LIMS for Induced Mutations by Sequencing and TILLing (V.G., E.C., unpublished). This web-based publicly accessible information system (http://limstill.niob.knaw.nl) was used to generate projects and visualize gene structures based on Ensembl genome data, the design of PCR primers, entry, archiving and primary interpretation of mutations. The primer design application within LIMSTILL is Primer3-based and parameters are set to design primers with an optimal melting temperature of 58°C. Predictions on the effect of amino acid changing mutations were also conducted within LIMSTILL and are based on stand-alone versions of two prediction programs: SIFT (Ng et al. (2003) Nucleic Acids Res. 31, 3812-14) and PolyPhen (Ramenskyet al. (2002) Nucleic Acids Res. 30, 3894-900). Calculations of introducing a premature stop codon with different mutation spectra are integrated into LIMSTILL and done for all 768 working amplicons.

### Results

### Effect of ENU on MSH6 knockout rat

The efficiency of ENU is dependent of the dose, reflected by a higher mutagenicity after three weekly administrations of low doses of the mutagen compared to a single high-dose injection (Justice et al. (2000) Mamm Genome 11, 484-8). Furthermore it has been shown that higher ENU concentrations increase the mutation frequency (Smits et al. (2004) Pharmacogenet Genomics 16, 159-69; Smits and Cuppen (2006) Trends Genet. 22, 232-40). To determine the optimal ENU concentration we performed an ENU dose-response experiment and determined the fertility of the founders. At 12 weeks of age *msh6*^{*-*/*-*} males were treated with 3 weekly doses of different concentrations of the mutagen. Fertility was determined after a full cycle of spermatogenesis (± 10 weeks) by breeding and/or histological analysis of the testes. At concentrations of 35, 30 and 25 mg/kg, respectively 1/8, 5/8 and 4/6 males were fertile (Table 5). Compared to earlier studies, where at 40 and 35 mg/kg ENU respectively 6/10 and 10/10 rats are fertile (Smits and Cuppen (2006) Trends Genet. 22, 232-40), this is a considerable decrease.
It is known that MSH6 deficiency reduces lifespan in both rats (van Boxtel et al. (2007) submitted) and mice (Edelmann (1997) Cell 91, 467-77) as a result of tumor development. MSH6 deficiency increased ENU sensitivity not only in fertility, but also in survival. Whereas untreated *msh6*^{*-*/*-*} rats show a median survival of 14 months, this is reduced in the ENU treated males to an average mean of 37 ± 3 weeks (∼ 8 months) and no male was alive after 50 weeks of age (Figure 5). This decrease in lifespan was due to the development of tumors, mainly lymphomas (data not shown). In previous studies, ENU treated wild-type males didn't show any reduced lifespan till 1,5 years of age in both 40 mg/kg bodyweight (10 out of 10 survived) and 35 mg/kg bodyweight (9 out of 10 survived) ENU treated males (unpublished data).

### Mutation resequencing protocol

Recently we have developed an automated, high-throughput and very cost-effective resequencing protocol using dideoxy sequencing (Smits et al. (2006) Pharmacogenet Genomics 16, 159-69). To reduce the costs even more, we designed a screening scheme, whereby the offspring of the mutagenized animals can be screened before weaning. As a result less animal-room space is needed increasing the cost-efficiency of the method.
At two weeks of age tissue samples from F1 offspring was taken and DNA was isolated (Figure 6A). Every pup was screened for 768 pre-selected amplicons by PCR amplifying and sequencing using two 384 wells plates that contained unique primers in every well (Figure 6B). The sequencing files of the same amplicons for different pups were aligned, processed using PolyPhred (Nickerson et al. (1997) Nucleic Acids Res. 25, 2745-51) and inspected with in house developed software (Figure 6C). Mutations were verified and interesting mutants were weaned (Figure 6D). This procedure was repeated for all new F1 offspring.

### Increased ENU mutagenicity in MSH6 deficient background

The optimal ENU treatment in MSH6 deficient background was 3 x 30 mg/kg bodyweight ENU in terms of fertility after a full cycle of spermatogenesis. It has been shown that in wild-type rats the highest possible dose, in terms of fertility, gave increased mutation frequencies (Smits et al. (2004) Genomics 83, 332-4; Smits et al. (2006) Pharmacogenet Genomics 25, 2745-51). After screening 291 F1 progeny of this group, covering almost 70 Mb, we discovered 96 ENU-induced mutations (Table 5). This results in mutation rate of 1 mutation every 7.26 x 10⁵ base pairs, a 1.7x increase compared to control animals.
Only one nest of three pups could be recovered from the only fertile male treated with 3 x 35 mg/kg bodyweight ENU. In the 0.72 Mb we sequenced, one mutation was discovered resulting in a comparable mutation rate as the 3 x 30 mg/kg treated group (Table 5). However the number is too low to conclude anything from this group. One male treated with 25 mg/kg bodyweight ENU produced 16 progeny that were screened. In this group 3.6 Mb was sequenced and 5 mutations were discovered, again resulting in a comparable mutation rate as observed in the 3 x 30 mg/kg treated group (Table 5).

### ENU-induced mutation frequency in MSH6 deficient background differs in time

Besides increasing the cost-efficiency, screening the F1 progeny before weaning also allows for mutation discovery in time. Offspring from the males treated with 3 x 30 mg/kg bodyweight ENU show a reduction of mutation frequency in time (Table 6). In the 59 animals that were born 14 - 16 weeks after the ENU injection we sequenced ±15 Mb and found 28 mutations, resulting in a mutation frequency of 1.88 x 10⁻⁶ per base pair and a rate of 1 mutation every 5.3 x 10⁵ base pairs. Animals that were born 17 - 19 weeks and 20 - 22 weeks after the last ENU injection show a decrease in mutation frequency of respectively 1.65 x 10⁻⁶ and 1.24 x 10⁻⁶ per base pair. F1 progeny that was born 23 weeks after the last injection or later show a steady frequency of approximately 1.0 x 10⁻⁶ per base pair. These last observations are accompanied by the high mortality rate in the founders (average mean of survival was 37 ± 3 weeks, which is 23 weeks after the last injection, Figure 5). Remarkably, all fertile 3 x 30 mg/kg bodyweight treated ENU males were still alive at this point. It is tempting to speculate whether there is a relationship between fertility and viability in these ENU treated MSH6 deficient males. However, the numbers of treated males are too low to make any conclusions about this remark.

### ENU-induced mutations and spectrum

In order to maximize the outcome of interesting mutants we mostly resequenced large exons of G protein-coupled receptors (GPCRs). It has been estimated that approximately 80% of all known hormones and neurotransmitters signal via GPCRs and that 30-45% of the current drug target these receptors (Gloriam et al. (2007) BMC Genomics 8, 338). Of the 102 mutations we have found, 94 are in coding regions (Table 7). Four mutations introduce a premature stop codon in the open reading frame and most likely will result in a functional knockout of that gene (nonsense, ± 4%), 70 mutations cause an amino acid change (missense, ± 74%) and 20 mutations do not effect protein coding (silent, ± 21%).
Three of the four new knockouts represent orphan GPCRs. In humans GPR19 encodes a gene that demonstrated closest similarity to D2 dopamine and neuropeptide Y receptors. Furthermore GPR19 expression significantly overlapped D2 dopamine receptor gene expression in peripheral and brain regions (O'Dowd et a1. (1996) FEBS Lett. 394, 325-9). Nothing much is know about GPR84 except that in humans is expressed in brain, heart, muscle, colon, thymus, spleen, kidney, liver, intestine, placenta, lung and leukocytes (Wittenberger et al. (2001) J Mol Biol. 307, 799-813). PSYR was designated T-cell death-associated gene-8 (Tdag8) in mice and demonstrated an elevated expression upon activation-induced apoptosis of T-cell hybridomas (Choi et al. (1996) Cell Immunol 168, 78-94). Finally CXCR2 encodes the interleukin 8 receptor, type 2 and was shown to be a major mediator of neutrophil migration to sites of inflammation (Cacalano et al. (1994) Science 265, 682-4). As in our previous studies (Smits et al, (2006) Pharmacogenet Genomics 16, 159-69), we used PolyPhen (Ramensky et al. (2002) Nucleic Acids Res. 30, 3894-900) and SIFT (Ng et al, (2003) Nucleic Acids Res. 31, 3812-4) software to predict the potential effect of the amino acid changes resulting from the missense mutations. Of the 70 missense mutations 30 were predicted to affect protein function by both programs (Table 3). Out of the remaining 40 missense mutations 30 were predicted to affect protein function by one of the two programs and 10 were predicted not to affect protein function by both programs (Table 7).
Remarkably, ENU-induces a different mutation spectrum under MSH6 deficient compared to MMR proficient background (Figure 7A). There is a significant difference between the spreads of the transitions under MSH6 proficient and deficient backgrounds (p = 0.028), but not between the transversions. The difference in mutation spectra most probably reflects MutSα mismatch binding preference. In *Lac*I mutational reporter studies it was shown that MSH6 deficient small intestinal epithelial cells of mice had a predominance of spontaneous G:C to A:T transitions (Mark et al. (2002) Oncogene 21, 7126-30). Furthermore *msh2*^{*-*/*-*} mouse ES cell treated with ENU show predominance for A:T to T:A transversions and G:C to A:T transitions (Claij et al, (2003) Cancer Res. 63, 2062-6). This change in mutation spectrum therefore underlines the specific effect ENU in MSH6 deficient background. The chance of introducing a stop codon depends on the mutation spectrum. This chance is unequally divided between the different mutation types. For example, G:C to A:T transitions can introduce 5 premature stop codons out of the 183 total changes in all possible codons, whereas A:T to G:C transitions will never lead to a premature stop codon. To determine the difference in chance of introducing a premature stop codon we calculated this chance for the 768 amplicons of the resequencing panel with the wild-type versus the mutant mutation spectra. The chance of introducing a premature stop codon with the wild-type mutation spectrum is ∼ 4.3%, whereas the chance using the *msh6*^{*-*/*-*} mutation spectrum is significantly (p << 0.01) elevated to ∼ 5.0% (Figure 7B). This means a 16% higher chance of introducing a premature stop under MSH6 deficient background compared to MSH6 proficient background.

**Table 1: MSI in the germ line of Msh6 deficient rats**

| **Father (genotype)** | **(CA)₃₆** | **(CA)₄₀** | **(G)₂₀** | **(A)₃₀** | **Pups tested** |
|---|---|---|---|---|---|
| **F (-/-)** | 1 | 3 | 2 | 0 | 17 |
| **G (-/-)** | 1 | 2 | 3 | 1 | 24 |
| **HA (-/-)** | 0 | 1 | 1 | 0 | 18 |
| **I (-/-)** | 0 | 1 | 2 | 0 | 9 |
| **J (-/-)** | 2 | 0 | 1 | 3 | 22 |
| **K (-/-)** | 0 | 2 | 1 | 0 | 14 |
| **L (-/-)** | 1 | 0 | 0 | 2 | 3 |
| **M (-/-)** | 0 | 2 | 1 | 0 | 14 |
| **N (-/-)** | 2 | 3 | 1 | 3 | 47 |
| **Average MSI (± SEM)** | 6% (± 4%) | 9% (± 2%) | 8% (± 2%) | 10% (± 7%) | Total pups tested = 168 |
| **Control (+/+) n = 19** | 0 | 0 | 0 | 0 | Total pups tested = 100 |

**Table 2: Spontaneous germ line mutation frequency**

| **Genotype Father** | **Total high quality bases sequenced** | **Number of verified mutations** | **Mutation frequency (per bp)** |
|---|---|---|---|
| **Msh6^{-/-} (n=2)** | 8,327,563 | 3 | 3.6 x 10⁻⁷ |
| **Msh6^{+/+} (n=4)** | 6,565,893 | 0 | < < 1.5 x 10⁻⁷ |

**Table 3: Tumor spectrum of the Msh6 knockout rat**

| **Rat ID (Genotype)** | **Gender** | **Age (months)** | **Tumor type** | **Tumor size¹** | **Involvement** |
|---|---|---|---|---|---|
| **17 (-/-)** | M | 18 | B-cell lymphoblastic lymphoma | ± 3 cm | Mediastinum |
| **25 (-/-)** | M | 11 | B-cell lymphoblastic lymphoma | N/D | Spleen, Liver, Kidney, Lung |
| **30 (-/-)** | F | 12 | B-cell lymphoblastic lymphoma | ± 3 cm | Mediastinum |
| **42 (-/-)** | M | 17 | B-cell lymphoblastic lymphoma | N/D | Bone marrow, mediastinum, liver |
| **47 (-/-)** | F | 15 | Squamous cell carcinoma | ± 2 cm | Stomach |
| **49 (-/-)** | F | 12 | Leiomyocarcinoma | ± 5 cm | Uterus |
| **52 (-/-)** | M | 18 | Leydig cell tumor | | Testis |
| **62 (-/-)** | M | 9 | B-cell lymphoblastic lymphoma | N/D | Spleen, Liver |
| **68 (-/-)** | F | 12 | Endometrial carcinoma | ± 1.5 cm | Uterus |
| **74 (-/-)** | F | 12 | Endometrial carcinoma | ± 1 cm | Uterus |
| **72 (-/-)** | F | 18 | B-cell lymphoblastic lymphoma | ± 1 cm | Mediastinum |
| **74 (-/-)** | M | 14 | Fibroadenoma and adenocarcinoma | ± 2 cm | Mammary gland |
| **81 (-/-)** | F | 18 | Endometrial carcinoma | ± 0.5 cm | Uterus |
| **85 (-/-)** | M | 17 | T-cell lymphoblastic lymphoma | ± 2 cm | Thymus |
| **89 (-/-)** | M | 10 | B-cell lymphoblastic lymphoma | N/D | Spleen, Liver |

| | | | | | |
|---|---|---|---|---|---|
| ¹Approximate tumor diameter estimated from slides | | | | | |

**Table 4: Comparison of rat and mouse msh6^{-/-} models with msh6 deficiency in human**

| | **Rat** | **Mouse** | **Human*** |
|---|---|---|---|
| **Occurrence of** | Yes | Yes | Yes |
| **genetic instability (e.g. MSI)** | (comparable numbers as in human or mouse) | (relatively low as compared to other MMR gene defects) | (relatively low as compared to other MMR gene defects) |
| **Onset tumorigenesis** | Relatively late in life | Relatively late in life | Relatively late in life (heterozygous mutations) ± 45 years |
| **Occurrence first tumors in population Median survival Tumor spectrum** | 9 months | 3 months | |
| | 14 months Lymphomas, tumors in the uterus, stomach, mammary gland, testes and leukemia | 9 months Lymphomas, tumors in the small intestine, liver, lung, skin, fibrous and endothelial cells | N/A Lymphomas (only in homozygous germ line mutations), tumors in the colon uterus, ovaries and stomach |
| **References** | | (Edelmann et al. (1997) Cell 91, 467-77; de Wind et al. (1999) Nat Genet 23, 359-62; Mark et al. (2002) Oncogene 21, 7126-30; Yang et al. (2004) Cancer Cell 6, 139-50) | (Wijnen et al. (1999) Nat Genet 23, 142-4; Wagner et al. (2001) J Med Genet 38, 318-22; Whiteside et al. (2002) Cancer Res 62, 359-62; |
| | | | Goodfellow et al. (2003) Proc Natl Acad Sci USA 100, 5908-13; |
| | | | Bandipalliam (2005) Fam Cancer 4 323-33) |

| | | | |
|---|---|---|---|
| * as observed in heterozygous Msh6 individuals, unless mentioned otherwise | | | |

**Table 5: Effect of different concentration of ENU**

| **Genotype/Dose*** | **MSH6^{-/-} 3 x 25** | **MSH6^{-/-} 3 x 30** | **MSH6^{-/-} 3x35** | **Total** | **Control 3 x 40^{†}** |
|---|---|---|---|---|---|
| **# Males injected** | 6 | 8 | 8 | 24 | 10 |
| **# Fertile males^{§}** | 4 | 5 | 1 | 10 | 6 |
| **# Pups for** | | | | | |
| **screening** | 16 | 291 | 3 | 310 | 362 |
| **# Bases** | 3.57 x 10⁶ | 69.66 x 10⁶ | 0.72 x 106 | 73,95 x 10⁶ | 37,27 x 10⁶ |
| **screened** | | | | | |
| **# Mutations** | | | | | |
| **- nonsense** | 0 | 4 | 0 | 4 | 0 |
| **- missense** | 4 | 65 | 1 | 70 | 17 |
| **- silent** | 1 | 19 | 0 | 20 | 7 |
| **- non-coding** | 0 | 8 | 0 | 8 | 6 |
| **-total** | 5 | 96 | 1 | 102 | 30 |
| **Mutation** | 1.40 x 10⁻⁶ | 1.38 x 10⁻⁶ | 1.38 x 10⁻⁶ | | 8.05 x 10⁻⁷ |
| **Frequencies** | | | | | |
| **Mutation Rates** | 1 in 7.14 x 10⁵ | 1 in 7.26 x 10⁵ | 1 in 7.22 x 10⁵ | | 1 in 1.24 x 10⁶ |

| | | | | | |
|---|---|---|---|---|---|
| *Dose is indicated as mg of ENU per kg bodyweight (three weekly injections) ^{†} Control 3 x 40 measurements were adapted from (Smits et al. (2006) Pramacogenet Genomics 16, 159-69) and represent the highest ENU-induced mutation frequency in rats ENU treated group ^{§} Fertility is measured by histological examination of the testes and/or the presence of at least one litter after 10 weeks after the last injection | | | | | |

**Table 6: ENU-induced mutation frequency differs in time**

| **Time of birth (weeks after lasts injection)** | **14 - 16** | **17 - 19** | **20 - 22** | **23 - 25** | **26 - 29** |
|---|---|---|---|---|---|
| **# Surviving founders** | 5/5 | 5/5 | 5/5 | 5/5 | 3/5 |
| **# Pups for screening** | 59 | 62 | 67 | 64 | 39 |
| **Average** | 12 ± 8 | 12 ± 11 | 13 ± 7 | 13 ± 9 | 13 ± 7 |
| **Pups/male** | | | | | |
| **# Bases screened** | 14.92 x 10⁶ | 13.30 x 106 | 15.34 x 10⁶ | 16.14 x 10⁶ | 9.97 x 10⁶ |
| **# Mutations** | 28 | 22 | 19 | 16 | 11 |
| **Mutation** | 1.88 x 10⁻⁶ | 1.65 x 10⁻⁶ | 1.24 x 10⁻⁶ | 9.92 x 10⁻⁷ | 1.10 x 10⁻⁶ |
| **Frequencies Mutation Rates** | | | | | |
| | 1 in 5.33 x 10⁵ | 1 in 6.05 x 10⁵ | 1 in 8.07 x 10⁵ | 1 in 10.08 x 10⁵ | 1 in 9.06 x 10⁵ |
| | | | | | |

**Table 7**

| **Gene** | **Description** | **ENSEMBL_ID*** | **Base change** | **AA Change** | **Pred. Cat.*** | **Domain information**** |
|---|---|---|---|---|---|---|
| ***Nonsense*** | ***mutations 4 (*± *4% of coding mutations)*** | | | | | |
| GPR19 | Probable G-protein coupled receptor 19 | ENSRNOG00000007126 | T17352A | L77X | - | truncation |
| GPR84 | Probable G-protein coupled receptor 84 | ENSRNOG00000036834 | T1057A | Y19X | - | truncation |
| PSYR | Psychosine receptor | ENSRNOG00000009922 | A1538T | K180X | - | truncation |
| CXCR2 | High affinity interleukin-8 receptor B | ENSRNOG00000014269 | T7103A | C307X | - | truncation |
| ***Missense mutations 70 (*± *74% of coding mutations)*** | | | | | | |
| 5HT2A | 5-hydroxytryptamine 2A receptor | ENSRNOG00000010063 | A1624G | N54D | 1 | Outside, before 1 tmd |
| AGTR1a | Type-1 anglotensin II receptor | ENSRNOG00000018346 | A54965T | D74V | 1 | In 2nd tmd |
| CX3C1 | CX3C chemokine receptor 1 | ENSRNOG00000018509 | T13800A | I118K | 1 | 3th tmd |
| DRD3 | D(3) dopamine receptor | ENSRNOG00000026772 | T73268C | S355P | 1 | Between 5th and 6th tmd |
| FZD9 | Frizzled 9 precursor | ENSRNOG00000001452 | A2521C | I428T | 1 | Between 5th and 6th tmd |
| GALR1 | Galanin receptor type 1 Gonadotropin-releasing hormone | ENSRNOG00000016654 | T1511A | Y95N | 1 | Between 2nd and 3th tmd |
| GNRHR | receptor | ENSRNOG00000002011 | T1365C | I93T | 1 | In 2nd tmd Between 5th and 6th |
| GP171 | Probable G-protein coupled receptor 171 | ENSRNOG00000025297 | A1507T | K169N | 1 | domain |
| GPR4 | Probable G-protein coupled receptor 4 | ENSRNOG00000016362 | T1862A | V190E | 1 | In 5th tmd |
| GPR85 | Probable G-protein coupled receptor 85 | ENSRNOG00000024636 | T2647A | V145E | 1 | In 4th tmd |
| MR | Mineralocorticoid receptor | ENSRNOG00000034007 | G2459T | G487C | 1 | N-terminus |
| piwil4 | piwi-like homolog 4 | ENSRNOG00000009043 | A2118G | D260G | 1 | Before PAZ domain |
| PTAFR | Platelet-activating factor receptor | ENSRNOG00000013231 | C5451T | R177W | 1 | In 2nd tmd |
| | | | C19651 | | | |
| SPR1 | G protein-coupled receptor 68 | ENSRNOG00000027863 | G | P71A | 1 | Between 5th and 6th tmd |
| DUFFY | Duffy antigen/chemokine receptor | ENSRNOG00000003365 | A1674T | Q225L | 1 | Ig-domain |
| GALR1 | Galanin receptor type 1 Probable G-protein coupled receptor 124 | ENSRNOG00000016654 | A27090T | I93F | 1 | In 3th tmd |
| GP124 | pre. | ENSRNOG00000012991 | T1566A | F113Y | 1 | In 4th tmd |
| MR | Mineralocorticoid receptor G-protein coupled receptor family C | ENSRNOG00000034007 | T1392C | V131A | 1 | N-terminus |
| GPC5D | group 5D | ENSRNOG00000008439 | A2592C | Q531P | 1 | 1 tmd |
| CXCR2 | High affinity Interleukin-8 receptor B | ENSRNOG00000014269 | A1595G | T199A | 1 | 7th tmd (?) |
| EDG2 | Lysophosphatidic acid receptor Edg-2 | ENSRNOG00000013656 | C7097A | H305Q | 1 | 7th tmd (?) |
| | | | T121158 | | | |
| FZD6 | Frizzled 6 precursor (Frizzled-6) | ENSRNOG00000004660 | G A10008 | M318R | 1 | 4th tmd |
| Cyp2D2 | Cytochrome P450 2D26 | ENSRNOG00000008988 | G | T101A | 1 | Cyt-P450 superfamily |
| MC5R | Melanocortin receptor 5 | ENSRNOG00000016685 | A3545T | M288L | 1 | 1st tmd |
| | | | T12564 | | | |
| RevErbA | Orphan nuclear receptor NR1D1 | ENSRNOG00000009329 | G | S574A | 1 | Serine-rich region profile |
| DUFFY | Duffy antigen/chemokine receptor | ENSRNOG00000003365 | A3908T | N38Y | 1 | In Ig-1 set |
| NPBW2 | Neuropeptides B/W receptor type 2 | ENSRNOG00000036716 | G8765A | D259N | 1 | 4th tmd |
| FZD9 | Frizzled 9 precursor | ENSRNOG00000001452 | A2671T | R478L | 2 | Between 6th and 7th tmd |
| 5HT1A | 5-hydroxytryptamine 1A receptor | ENSRNOG00000010254 | A36722T | I924F | 2 | Between 5th and 6th tmd |
| C3AR | C3a anaphylatoxin chemotactic receptor | ENSRNOG00000009211 | T1884A | V295E | 2 | Between 4th and 5th tmd |
| C3AR | C3a anaphylatoxin chemotactic receptor | ENSRNOG00000009211 | A6661T | D177V | 2 | Between 4th and 5th tmd |
| | | | | | | Corticosteroid-binding |
| CBG | Corticosteroid-binding globulin precursor | ENSRNOG00000009438 | C6667T | S179F | 2 | globulin domain |
| DICER | Dicer1 protein | ENSRNOG00000010711 | A3625T | S23C | 2 | In Helicase C domain |
| | Rat FERM- and PDZ-domain containing | | | | | |
| FRMPDlr | gene 1 | ENSRNOG00000012546 | T20036C | V535A | 2 | Not annotated |
| | | | A56703 | | | |
| GPR87 | Probable G-protein coupled receptor 87 | ENSRNOG00000013894 | G | D928G | 2 | Before 1st tmd |
| | Melanin-concentrating hormone receptor | | | | | |
| MCHR1 | 1 | ENSRNOG00000018895 | C5690T | P46L | 2 | Between 1st and 2nd tmd |
| MTR1B | Melatonin receptor type 1B | ENSRNOG00000008972 | T2682A | L138Q | 2 | In 6th tmd |
| | | | G38164 | | | |
| MTR1L | Melatonin-related receptor | ENSRNOG00000011335 | A | A255T | 2 | After 7th tmd |
| OX2R | Orexin receptor type 2 | ENSRNOG00000011251 | C4757A | P386H | 2 | Between 2nd and 3th tmd |
| PI2R PPAR- alpha | Prostacyclin receptor Peroxisome proliferator-activated receptor a | ENSRNOG00000016756 | T86127A | F122L | 2 | Between 1st and 2nd tmd |
| | | ENSRNOG00000021463 | C1293T | R71W | 2 | 48508 superfamily domain |
| PPAR- | Peroxisome proliferator-activated | | | | | |
| alpha | receptor a | ENSRNOG00000021463 | T52986A | F218Y | 2 | 57716 superfamily domain |
| PPAR- | Peroxisome proliferator-activated | | | | | Hormone receptor ligand |
| alpha | receptor a | ENSRNOG00000021463 | T52896A | I188N | 2 | binding domain |
| | | | G75161 | | | |
| Q6ZN22 | Vasoactive intestinal peptide receptor 2 | ENSRNOG00000004317 | A | V306I | 2 | In 6th tmd |
| | Mu opioid receptor splice variant hMOR- | | | | | Between 5th and 6th |
| Q86V80 | 1B3 | ENSRNOG00000018191 | T66496C | F336L | 2 | domain |
| VMT2 | Synaptic Vesicle Amine Transporter | ENSRNOG00000008890 | A34315T | M281L | 2 | In 2nd tmd |
| | Follicle-stimulating hormone receptor | | T12046 | | | One amino acid befor 4th |
| FSHR | pre | ENSRNOG00000016783 | G | F164V | 2 | tmd |
| GP151 | Probable G-protein coupled receptor 151 | ENSRNOG00000027103 | A61281T | R233S | 2 | Signal peptide? |
| GPR84 | Probable G-protein coupled receptor 84 | ENSRNOG00000036834 | T1040A | S14T | 2 | Between 3th and 4th tmd |
| GPR87 | Probable G-protein coupled receptor 87 | ENSRNOG00000013894 | G1052A | V18I | 2 | 6th tmd |
| Cyp2A2 | Cytochrome P450 2A2 | ENSRNOG00000020817 | C31716T | T300M | 2 | Not annotated |
| FZD7 | Frizzled 7 precursor (Frizzled-7) | ENSRNOG00000016119 | T1799A | V234E | 2 | 4th tmd |
| | | | T235491 | | | |
| ACTHR | Adrenocorticotropic hormone receptor | ENSRNOG00000016681 | C | V488A | 3 | After 7th tmd E-class P450 group I |
| Cyp2c12 | Cytochrome P450 2C40 | ENSRNOG00000012896 | T1874A | F292I | 3 | domain |
| | | | A42456 | | | Inside between 1st and |
| Cyp2D3 | Cytochrome P450 2D3 | ENSRNOG00000029179 | G | M339V | 3 | 2nd tmd |
| DICER | Dicer1 protein High affinity immunoglobulin epsilon | ENSRNOG00000010711 | A2790T | T183S | 3 | Rnase domain |
| FceR1a | receptor | ENSRNOG00000009177 | T37824A | I1593N | 3 | In Ig_sub domain |
| GPR12 | Probable G-protein coupled receptor 12 | ENSRNOG00000039832 | T3048A | I37N | 3 | In 3th tmd |
| IGE | Ig epsilon chain C region | ENSRNOG00000005328 | A1529T | M177L | 3 | Ig-domain |
| MC5R | Melanocortin receptor 5 | ENSRNOG00000016685 | A5516G | I74V | 3 | In 7th tmd |
| P34969 | 5-hydroxytryptamine 7 receptor | ENSRNOG00000018827 | A2878T | I317L | 3 | Between 3th and 4th tmd |
| | | | A115582 | | | |
| SGII | Secretogranin-II | ENSRNOG00000015055 | T | T192S | 3 | After 1st tmd outside |
| SUCR1 | Succinate receptor 1 | ENSRNOG00000014039 | A5768T | M561L | 3 | Between 4th and 5th tmd |
| | | | | | | Between 5th and 6th |
| DRD2 | D(2) dopamine receptor | ENSRNOG00000008428 | G1491A | G164S | 3 | domain |
| GASR | Gastrin/cholecystokinin type B receptor | ENSRNOG00000017679 | T1113C | I38T | 3 | Not annotated |
| MC4R | Melanocortin receptor 4 | ENSRNOG00000018692 | C9850A | L302I | 3 | 5th tmd |
| | | | A24961 | | | |
| G109B | Nicotinic acid receptor 1 | ENSRNOG00000026653 | G | T339A | 3 | After 7th tmd |
| GPR35 | Highly similar to Probable GPR35 Monogenic audiogenic seizure | ENSRNOG00000024030 | C2067T | A356V | 3 | Signal peptide |
| MASS1 | susceptibility p1 | ENSRNOG00000016306 | C6316T | R255C | 3 | Calx_beta domain |
| PINK1 | PINK1 | ENSRNOG00000015385 | T2198A | I90N | 3 | Not annotated |
| ***Silent mutations 20 (*± 21% *of coding mutations)*** | | | | | | |
| ***Non-coding region 8*** | | | | | | |
| **Total mutations 102 (of which 94 in coding regions)** | | | | | | |

| | | | | | | |
|---|---|---|---|---|---|---|
| * Ensembl ID's are numbers as indicated in database version 47.34q | | | | | | |

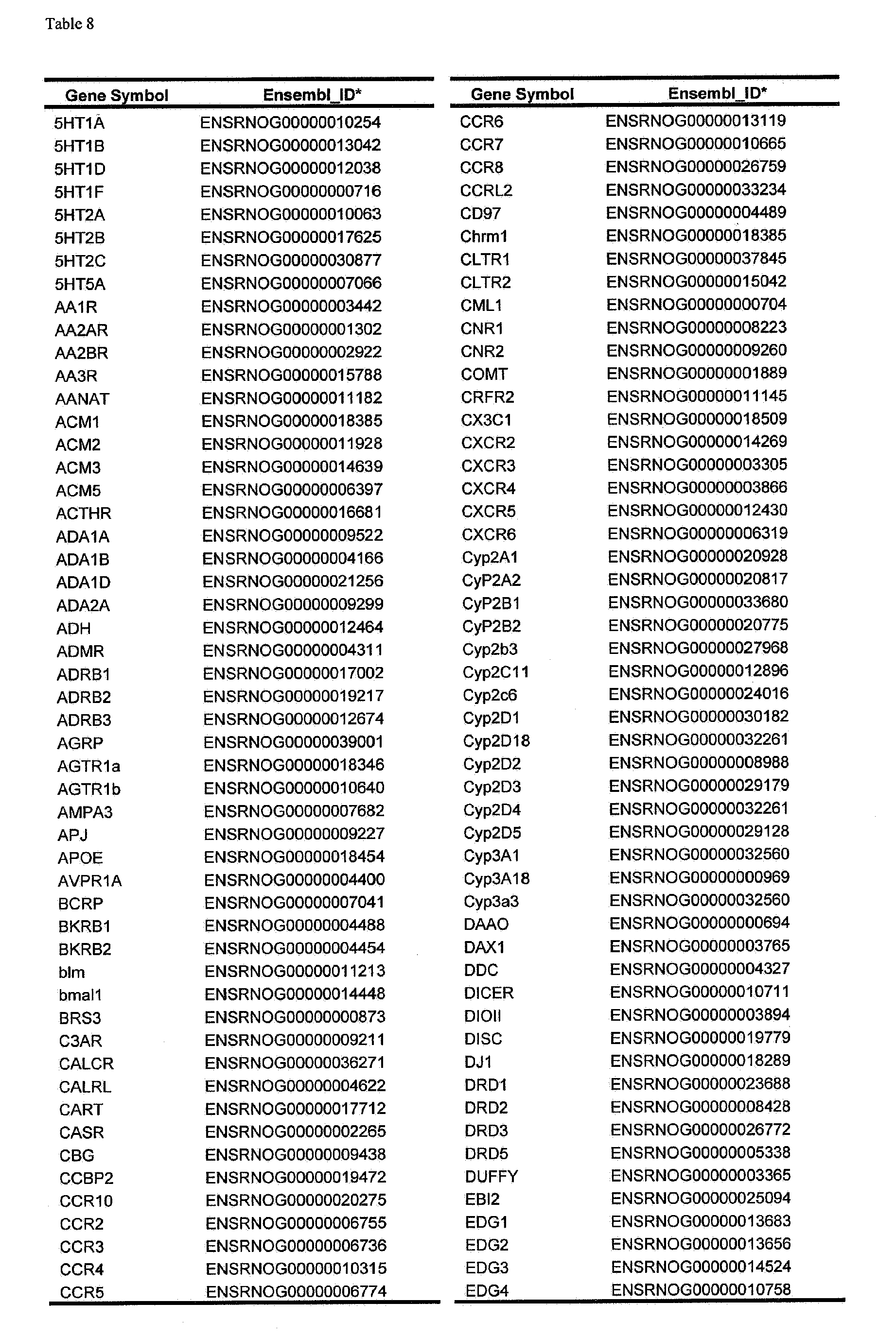

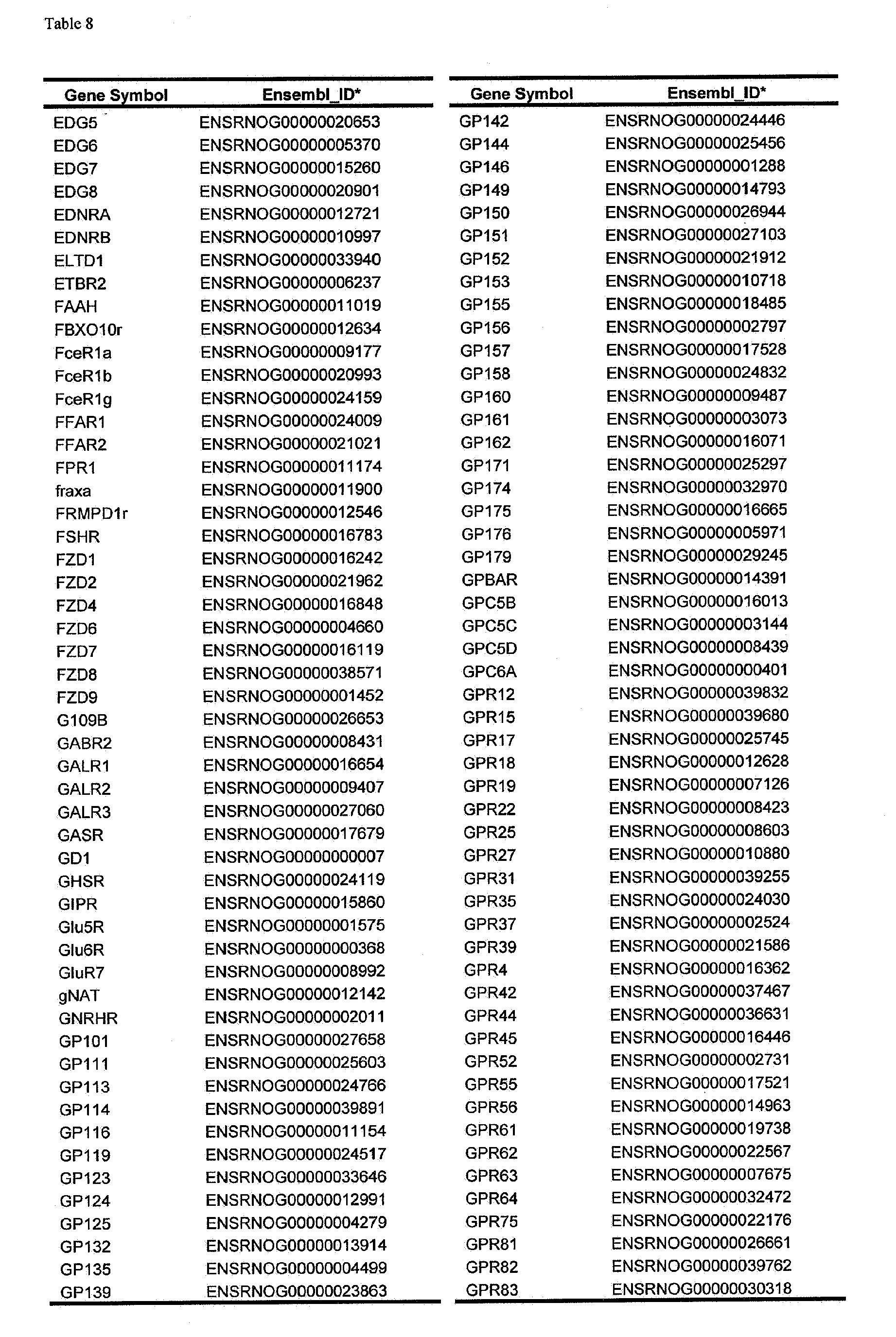

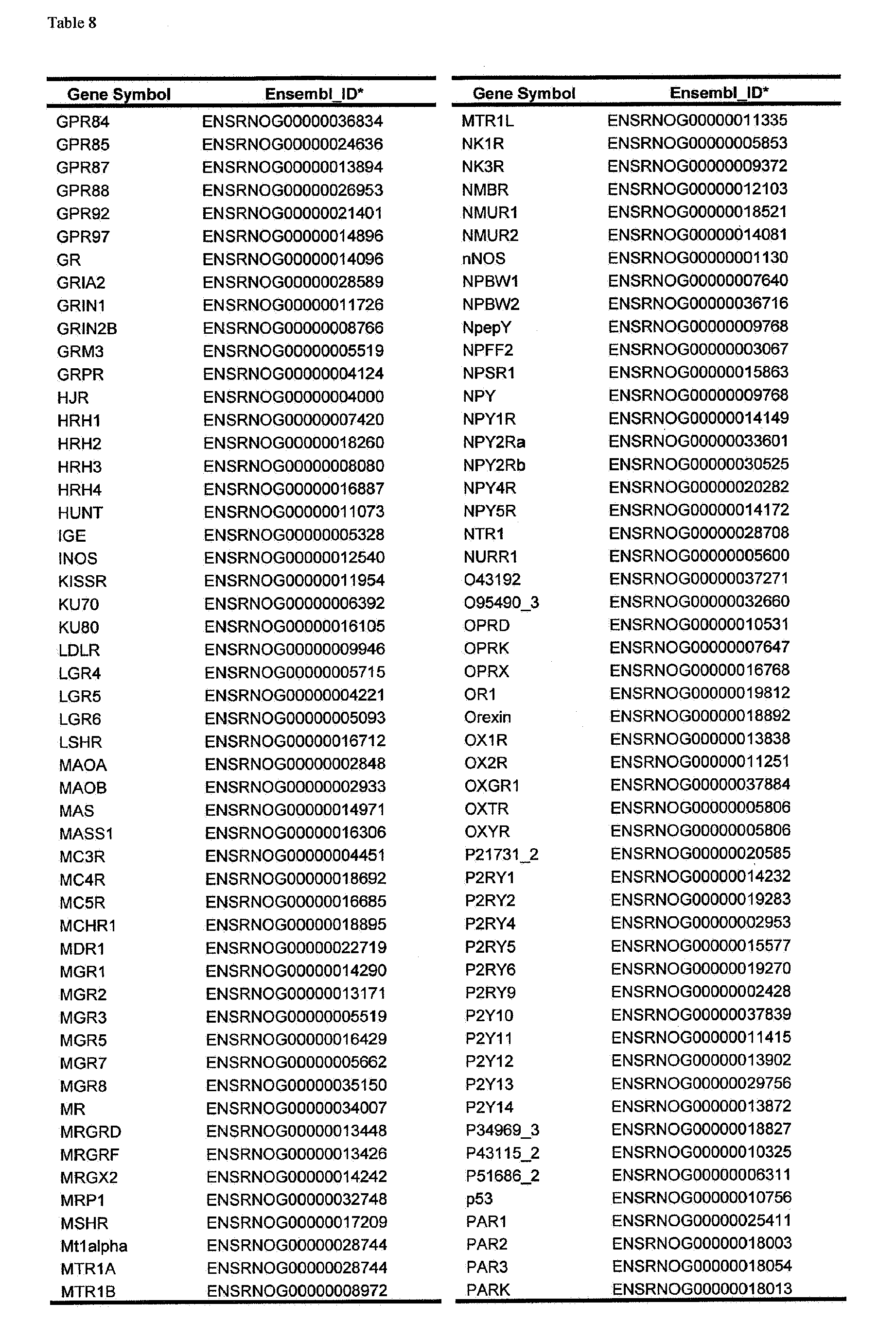

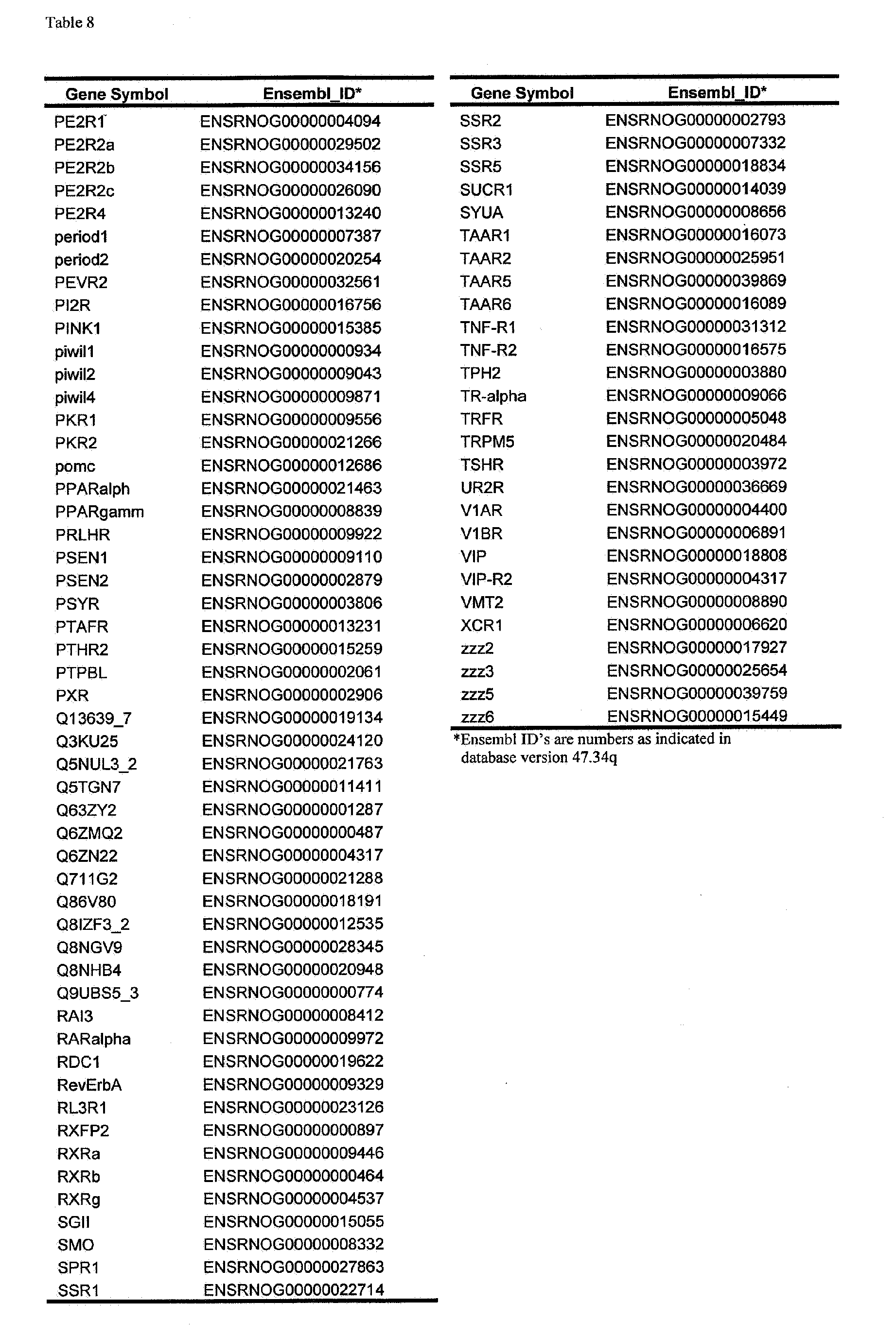

## Claims

1. A method for producing an animal of the genus rattus with a mutagen induced mutation in a germ line cell comprising providing said animal having said germ line cell with a mutation inducing amount of a mutagen, said method **characterised in that** msh6 expression in said germ line cell is functionally decreased.

2. A method according to claim 1, wherein said germ line cell comprises a genetic modification resulting in said functionally decreased expression of Msh6.

3. A method according to claim 1 or claim 2, wherein essentially all cells of said animal comprise said functionally decreased msh6 expression.

4. A method according to any one of claims 1-3, further comprising generating offspring with said germ line cell, a matured derivative thereof or a nucleus thereof.

5. A method according to any of claims 1-4, further comprising collecting germ line cells from said animal that has been contacted with said mutagen.

6. A method according to claim 5, further comprising generating offspring with said collected germ line cells.

7. A method according to claim 4 or claim 6, further comprising collecting and storing cells of said offspring and/or nucleic acid of said cells.

8. A method according to claim 7, wherein said cells of said offspring comprise a germ line cell or derivative thereof.

9. A method according to any one of claims 1-8, further comprising screening offspring of said animal for the presence of a mutation in a specific nucleic acid sequence.

10. A method according to claim 9, wherein said screening comprises the detection of a specific sequence motif.

11. A method according to claim 10, wherein said motif comprises a stopcodon.

12. A method according to any one of claims 2-11, further comprising breeding said offspring animals to segregate said mutagen induced mutation from a genetic modification that causes said functionally decreased Msh6 expression.

13. An animal of the genus rattus comprising a cell with a mutagen induced mutation **characterised in that** Msh6 expression in said cell is functionally decreased.

14. An animal according to claim 13, wherein essentially all cells of said animal comprise a functionally decreased Msh6 expression.

15. An animal with a germ line cell that comprises at least one mutagen induced mutation in every 0.1 x 10⁶ to 1 x 10⁶ base pairs.

16. An animal according to claim 15, wherein germ lines cells of said animal comprise on average at least one mutagen induced mutation in every 1 x 10⁶ base pairs, preferably at least 1 mutagen induced mutation in every 0.5 x 10⁶ base pairs.

17. An animal according to claim 15 or claim 16, wherein essentially all cells of said animal comprise a functionally decreased Msh6 expression.

18. An animal of the genus rattus, having a mutation in a gene as listed in table 7 or table 8.

19. Progeny of an animal according to claim 18, having a mutation in a gene as listed in table 7 or table 8, and comprising cells having functional msh6 expression.

20. Progeny according to claim 18 or claim 19, that are homozygous for said mutation in a gene listed in table 7 or table 8.

21. An animal of the genus rattus comprising a cell with a mutagen induced mutation that is a progeny of an animal according to any one of claims 13-20.

22. An animal according to claim 21, comprising a cell wherein msh6 expression is functionally decreased.

23. A collection of germ-line cells or derivatives thereof of an animal according to any one of claims 13-22.

24. A storage means comprising a collection of germ-line cells according to claim 23.

25. A storage means according to claim 24, comprising germ-lines cells of individual animals according to any one of claims 13-22, in separate containers.

26. Use of an animal of the genus rattus comprising cells wherein msh6 expression is functionally decreased for generating an animal of the genus rattus having a mutation in a gene other than msh6.

27. Use of an animal according to any one of claims 13-22, for generating a rat having a mutation in a gene other than msh6.

28. Use according to claim 26 or claim 27, wherein said mutation is mutagen induced.

29. Use of an animal of the genus rattus in which Msh6 expression is functionally decreased as an animal model for HNPCC.

30. Use according to claim 29, for testing a potential pharmaceutical therapeutic agent.
